(19) Europäisches Patentamt / European Patent Office / Office européen des brevets

(11) **EP 4 740 924 A1**

(12) **EUROPEAN PATENT APPLICATION**
published in accordance with Art. 153(4) EPC

(43) Date of publication:
**13.05.2026 Bulletin 2026/20**

(21) Application number: **24897848.8**

(22) Date of filing: **27.09.2024**

(51) International Patent Classification (IPC):
*A61H 3/00* (2006.01)    *B25J 9/00* (2006.01)
*G01R 31/28* (2006.01)    *G01R 19/165* (2006.01)
*G01R 31/34* (2020.01)    *G01R 31/52* (2020.01)
*G08C 17/02* (2006.01)    *H05B 45/10* (2020.01)

(52) Cooperative Patent Classification (CPC):
A61H 3/00; B25J 9/00; G01R 19/165; G01R 31/28;
G01R 31/34; G01R 31/52; G08C 17/02;
H05B 45/10

(86) International application number:
**PCT/KR2024/014703**

(87) International publication number:
**WO 2025/116249 (05.06.2025 Gazette 2025/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.11.2023 KR 20230168244**

(71) Applicant: **Samsung Electronics Co., Ltd.
Suwon-si, Gyeonggi-do 16677 (KR)**

(72) Inventor: **PARK, Jihye
Suwon-si Gyeonggi-do 16677 (KR)**

(74) Representative: **Gulde & Partner
Patent- und Rechtsanwaltskanzlei mbB
Berliner Freiheit 2
10785 Berlin (DE)**

(54) **WEARABLE DEVICE FOR PERFORMING DIAGNOSTIC OPERATION, AND OPERATING METHOD THEREOF**

(57)    This wearable device can generate a second voltage on the basis of a first voltage used by a motor, consume, through a discharge circuit including one or more resistors, electromotive force generated by the motor if the second voltage is greater than or equal to a reference voltage, output a diagnostic voltage generated on the basis of the first voltage, the resistor in the discharge circuit, and one or more electrical elements, and determine whether the discharge circuit is in an abnormal state on the basis of at least one from among the voltage value of the second voltage or the voltage value of the diagnostic voltage.

FIG. 7

**Description**

**TECHNICAL FIELD**

Certain example embodiments relate to a wearable apparatus performing a diagnostic operation and/or an operating method of the wearable apparatus.

**BACKGROUND ART**

As society ages, more people are experiencing discomfort and pain while walking due to muscle weakness or joint issues caused by aging or the like. This has led to increased interest in walking assistance devices that may help elderly or other individuals with weakened muscles or patients with joint discomfort walk more smoothly and/or to exercise.

**DISCLOSURE OF THE INVENTION**

**TECHNICAL GOALS**

According to an example embodiment, a wearable apparatus may include a motor, a conversion circuit configured to generate a second voltage based on a first voltage used by the motor, a discharging circuit including one or more resistors and configured to consume an electromotive force generated by the motor through the one or more resistors when the second voltage is greater than or equal to a reference voltage, a diagnostic circuit including one or more electrical circuit elements (e.g., resistor(s)) and configured to output a diagnostic voltage generated based on the first voltage, a resistor in the discharging circuit, and the one or more electrical elements, and at least one processor. The processor(s), comprising processing circuitry, may be individually and/or collectively configured to receive the second voltage from the conversion circuit and receive the output diagnostic voltage from the diagnostic circuit. The processor(s) may be configured to determine whether the discharging circuit is in an abnormal state based on at least one of a voltage value of the second voltage or a voltage value of the received diagnostic voltage.

According to an example embodiment, a wearable apparatus may include a motor, a first circuit including one or more electrical circuit elements and configured to determine whether the motor is in an overvoltage state based on a first voltage used by the motor, a second circuit including one or more resistors and configured to consume an electromotive force generated by the motor through the one or more resistors, and at least one processor comprising processing circuitry. The first circuit may be configured to output a second voltage generated based on the first voltage to the processor and output a diagnostic voltage generated based on the first voltage, a resistor in the second circuit, and the one or more electrical elements to the processor. The processor(s) may be individually and/or collectively configured to determine whether the second circuit is in an abnormal state based on at least one of a voltage value of the second voltage or a voltage value of the diagnostic voltage.

According to an example embodiment, an operating method of a wearable apparatus may include generating a second voltage based on a first voltage used by a motor of the wearable apparatus, consuming an electromotive force generated by the motor through a discharging circuit including one or more resistors when the second voltage is greater than or equal to a reference voltage, generating a diagnostic voltage based on the first voltage, a resistor in the discharging circuit, and one or more electrical elements, and determining whether the discharging circuit is in an abnormal state based on at least one of a voltage value of the second voltage or a voltage value of the diagnostic voltage.

According to an example embodiment, when an excessive electromotive force is generated by a motor, a wearable apparatus may consume the excessive electromotive force as thermal energy through a discharging circuit (e.g., one or more resistors in the discharging circuit).

According to an example embodiment, a wearable apparatus in a pre-exercise state, an exercising state, or a post-exercise state may diagnose or inspect a discharging circuit according to a user request or periodically.

According to an example embodiment, a wearable apparatus may perform different diagnostic operations for a discharging circuit according to the amount of generated electromotive force of a motor, thereby reducing the amount of battery power that may be consumed when the discharging circuit is diagnosed.

**BRIEF DESCRIPTION OF DRAWINGS**

The above and other aspects, features, and advantages of certain example embodiments of the present disclosure will be more apparent from the following detailed description, taken in conjunction with the accompanying drawings, in which:

FIG. 1 is a diagram illustrating an overview of a wearable apparatus worn on a body of a user, according to an example embodiment.

FIG. 2 is a diagram illustrating an exercise management system including a wearable apparatus and an electronic device, according to an example embodiment.

FIG. 3 is a rear schematic view of a wearable apparatus according to an example embodiment.

FIG. 4 is a left side view of a wearable apparatus according to an example embodiment.

FIGS. 5A and 5B are diagrams illustrating a configuration of a control system of a wearable apparatus, according to an example embodiment.

FIG. 6 is a diagram illustrating an interaction between a wearable apparatus and an electronic device, according to an example embodiment.

FIG. 7 is a block diagram illustrating an example of a configuration of a wearable apparatus, according to an example embodiment.

FIG. 8 is a diagram illustrating an example of a conversion circuit, a comparison circuit, a control circuit, a discharging circuit, a diagnostic circuit, a first switch circuit, and a second switch circuit of a wearable apparatus, according to an example embodiment.

FIG. 9 is a diagram illustrating an example of a diagnostic operation of a wearable apparatus, according to an example embodiment.

FIGS. 10 and 11 are diagrams illustrating examples of a diagnostic operation of a wearable apparatus in a state in which an electromotive force of a motor of the wearable apparatus is not consumed, according to an example embodiment.

FIGS. 12, 13, and 14 are diagrams illustrating examples of a diagnostic operation of a wearable apparatus in a state in which an electromotive force of a motor of the wearable apparatus is consumed, according to an example embodiment.

FIG. 15 is a flowchart illustrating an example of a diagnostic operation when a wearable apparatus is in a pre-exercise state or a post-exercise state of a user, according to an example embodiment.

FIG. 16 is a flowchart illustrating an example of a diagnostic operation when a wearable apparatus is in an exercising state of a user, according to an example embodiment; and

FIG. 17 is a block diagram illustrating an example of a configuration of a wearable apparatus, according to an example embodiment.

## BEST MODE FOR CARRYING OUT THE INVENTION

The following detailed structural or functional description is provided as an example only and various alterations and modifications may be made to example embodiments. Here, the examples are not construed as limited to the disclosure and should be understood to include all changes, equivalents, and replacements within the idea and the technical scope of the disclosure.

Although terms, such as first, second, and the like are used to describe various components, the components are not limited to the terms. These terms should be used only to distinguish one component from another component. For example, a first component may be referred to as a second component, and similarly the second component may also be referred to as the first component.

It should be noted that if it is described that one component is "connected", "coupled", or "joined" to another component, at least a third component may be "connected", "coupled", and "joined" between the first and second components, although the first component may be directly connected, coupled, or joined to the second component. Thus, for example, "connected" covers both direct and indirect connections.

The singular forms "a", "an", and "the" include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms used herein including technical or scientific terms have the same meaning as commonly understood by one of ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, certain example embodiments will be described in detail with reference to the accompanying drawings. When describing the embodiments with reference to the accompanying drawings, like reference numerals refer to like elements and a repeated description related thereto will be omitted.

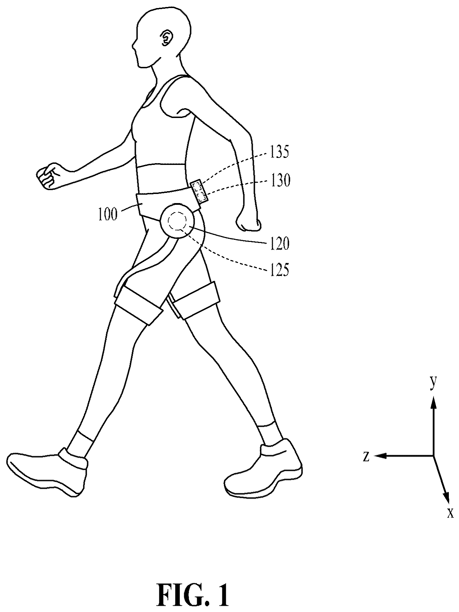

FIG. 1 is a diagram illustrating an overview of a wearable apparatus worn on a body of a user, according to an embodiment.

Referring to FIG. 1, in an embodiment, a wearable apparatus 100 may be a device worn on the body of a user to assist the user in walking, exercising, and/or working. In an embodiment, the wearable apparatus 100 may be used to measure a physical ability (e.g., a walking ability, an exercise ability, and an exercise posture) of the user. In embodiments, the term "wearable apparatus" may be replaced by "wearable robot", "walking assistance device", or "exercise assistance device". The user may be a human or an animal, and embodiments are not limited thereto. The wearable apparatus 100 may be worn on the body (e.g., the lower body (the legs, ankles, knees, etc.), the upper body (the torso, arms, wrists, etc.), or the waist) of the user to provide an external force such as an assistance force and/or a resistance force to a body motion of the user. The assistance force may be a force applied in the same direction as the body motion direction of the user and assisting the body motion of the user. The resistance force may be a force applied in a direction opposite to the body motion direction of the user and hindering the body motion of the user. The term "resistance force" may also be referred to as "exercise load".

In an embodiment, the wearable apparatus 100 may operate in a walking assistance mode for assisting the user in walking. In the walking assistance mode, the wearable apparatus 100 may assist the user in walking by applying an assistance force generated by a driving module 120 of the wearable apparatus 100 to the body of the user. The wearable apparatus 100 may enable the user to walk independently or to walk for a long time by providing a force required for the user to walk, thereby extending the walking ability of the user. The wearable apparatus 100 may help improve an abnormal walking habit or gait posture of a walker.

In an embodiment, the wearable apparatus 100 may operate in an exercise assistance mode (or a resistance mode) for enhancing the exercise effect of the user. In the exercise assistance mode (or the resistance mode), the wearable apparatus 100 may hinder the body motion of the user or provide resistance to the body motion of the user by applying a resistance force generated by the driving module 120 to the body of the user. When the wearable apparatus 100 is a hip-type wearable apparatus that is worn on the waist (or pelvis) and legs (e.g., thighs) of the user, the wearable apparatus 100 may provide an exercise load to a leg motion of the user while being worn on the legs, thereby enhancing the exercise effect on the legs of the user. In an embodiment, the wearable apparatus 100 may apply an assistance force to the body of the user to assist the user in exercising. For example, when a handicapped person or an elderly person wants to exercise wearing the wearable apparatus 100, the wearable apparatus 100 may provide an assistance force for assisting the body motion during the exercise process. In an embodiment, the wearable apparatus 100 may provide an assistance force and a resistance force in combination for each exercise section or time section, in such a manner of providing an assistance force in some exercise sections and a resistance force in other exercise sections.

In an embodiment, the wearable apparatus 100 may operate in a physical ability measurement mode for measuring a physical ability of the user. The wearable apparatus 100 may measure motion information of the user using sensors (e.g., an angle sensor 125 and an inertial measurement unit (IMU) 135) provided in the wearable apparatus 100 while the user is walking or exercising and may evaluate the physical ability of the user based on the measured motion information. For example, a gait index or an exercise ability index (e.g., the muscular strength, endurance, balance, or exercise posture) of the user may be estimated through the motion information of the user measured by the wearable apparatus 100. The physical ability measurement mode may include an exercise posture measurement mode for measuring an exercise posture of the user.

In various embodiments of the present disclosure, for convenience of description, the wearable apparatus 100 is described as an example of a hip-type wearable apparatus, as illustrated in FIG. 1, but the embodiments are not limited thereto. As described above, the wearable apparatus 100 may be worn on a body part (e.g., the upper arms, lower arms, hands, calves, and feet) other than the waist and legs (particularly, the thighs), and the shape and configuration of the wearable apparatus 100 may vary depending on the body part on which the wearable apparatus 100 is worn.

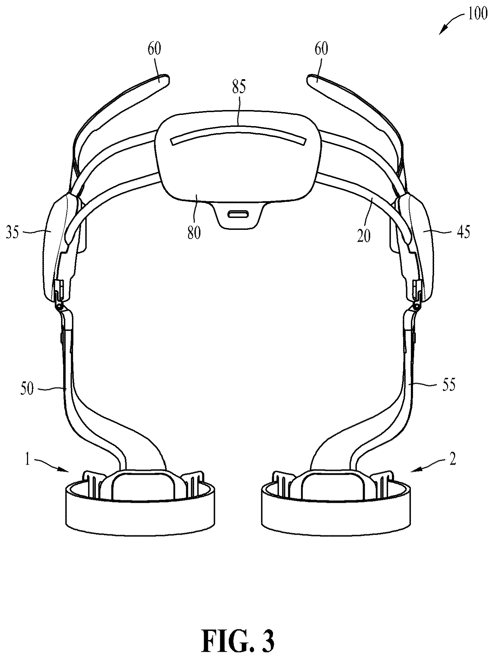

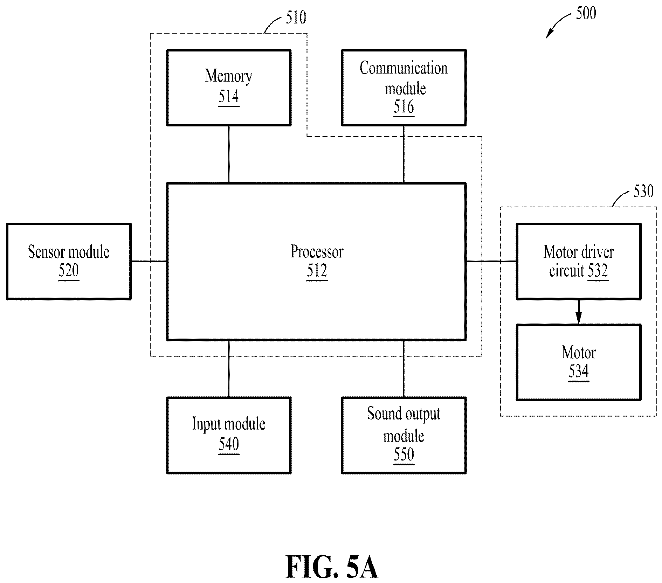

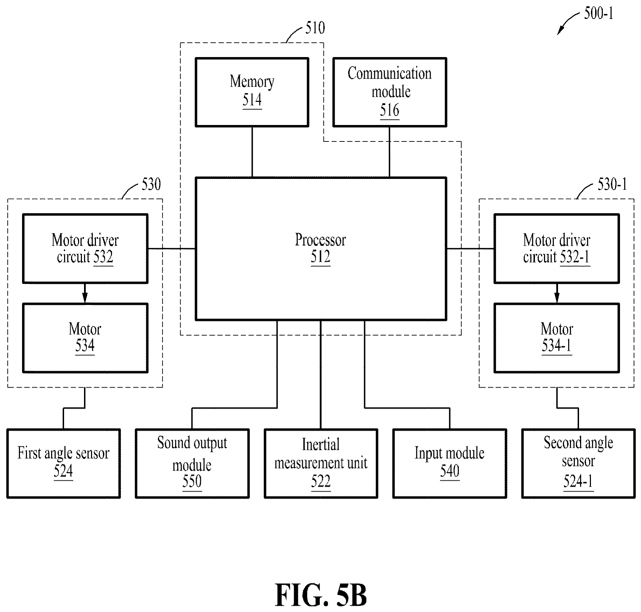

According to an embodiment, the wearable apparatus 100 may include a support frame (e.g., leg support frames 50 and 55 and a waist support frame 20 of FIG. 3) configured to support the body of the user when the wearable apparatus 100 is worn on the body of the user, a sensor module (e.g., a sensor module 520 of FIG. 5A comprising at least one sensor) configured to obtain sensor data including motion information about the body motion (e.g., a leg motion and an upper body motion) of the user, the driving module 120 (e.g., driving modules 35 and 45 of FIG. 3) configured to generate a torque to be applied to the legs of the user, and a control module 130 (e.g., a control module 510 of FIGS. 5A and 5B, comprising processing circuitry) configured to control the wearable apparatus 100.

The sensor module may include the angle sensor 125 and the IMU 135. The angle sensor 125 may measure the rotational angle of a leg support frame of the wearable apparatus 100 corresponding to the hip joint angle value of the user. The rotational angle of the leg support frame measured by the angle sensor 125 may be estimated as the hip joint angle value (or leg angle value) of the user. The angle sensor 125 may include, for example, an encoder and/or a Hall sensor. In an embodiment, the angle sensor 125 may be present near each of the right hip joint and the left hip joint of the user. The IMU 135 may include an acceleration sensor and/or an angular velocity sensor and may measure a change in acceleration and/or angular velocity according to the motion of the user. The IMU 135 may measure, for example, an upper body motion value of the user corresponding to a motion value of a waist support frame (or a base body (a base body 80 of FIG. 3)) of the wearable apparatus 100. The motion value of the waist support frame measured by the IMU 135 may be estimated as the upper body motion value of the user.

In an embodiment, the control module 130 and the IMU 135 may be arranged within a base body (e.g., the base body 80 of FIG. 3) of the wearable apparatus 100. The base body may be positioned on the lumbar region (an area of the lower back) of the user while the user is wearing the wearable apparatus 100. The base body may be formed or attached to the outer side of the waist support frame of the wearable apparatus 100. The base body may be mounted on the lumbar region of the user to provide a cushioning feeling to the lower back of the user and may support the lower back of the user together with the waist support frame.

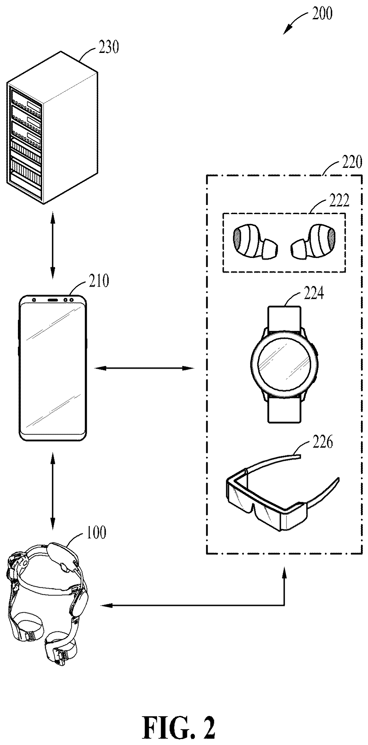

FIG. 2 is a diagram illustrating an exercise management system including a wearable apparatus and an electronic device, according to an embodiment.

Referring to FIG. 2, an exercise management system 200 may include the wearable apparatus 100, an electronic device 210, another wearable apparatus 220, and a server 230. In an embodiment, at least one (e.g., the other wearable apparatus 220 or the server 230) of these devices may be omitted from the exercise management system 200, or one or more other devices (e.g., an exclusive controller device of the wearable apparatus 100) may be added to the exercise management system 200.

In an embodiment, the wearable apparatus 100 may be worn on the body of the user in the walking assistance mode to assist the motion of the user. For example, the wearable apparatus 100 may be worn on the legs of the user to help the user in walking by generating an assistance force for assisting a leg motion of the user.

In an embodiment, the wearable apparatus 100 may generate a resistance force for hindering the body motion of the user or an assistance force for assisting the body motion of the user and apply the generated resistance force or assistance force to the body of the user to enhance the exercise effect of the user in an exercise assistance mode (or a resistance mode). In the exercise assistance mode, the user may select, through the electronic device 210, an exercise program (e.g., squat, split lunge, dumbbell squat, lunge and knee up, stretching, or the like) to perform using the wearable apparatus 100 and/or an exercise intensity to be applied to the wearable apparatus 100. The wearable apparatus 100 may control a driving module of the wearable apparatus 100 according to the exercise program selected by the user and obtain sensor data including motion information of the user through a sensor module. The wearable apparatus 100 may adjust the strength of the resistance force or assistance force applied to the user according to the exercise intensity selected by the user. For example, the wearable apparatus 100 may control the driving module to generate a resistance force corresponding to the exercise intensity selected by the user.

In an embodiment, the wearable apparatus 100 may be used to measure a physical ability of the user in interoperation with the electronic device 210. The wearable apparatus 100 may operate in a physical ability measurement mode, which is a mode for measuring a physical ability of the user, under the control by the electronic device 210, and may transmit sensor data obtained by the motion of the user in the physical ability measurement mode to the electronic device 210. The electronic device 210 may estimate the physical ability of the user by analyzing the sensor data received from the wearable apparatus 100.

The electronic device 210 may communicate with the wearable apparatus 100 and may remotely control the wearable apparatus 100 or provide the user with state information about a state (e.g., a booting state, a charging state, a sensing state, or an error state) of the wearable apparatus 100. The electronic device 210 may receive the sensor data obtained by a sensor in the wearable apparatus 100 from the wearable apparatus 100 and estimate the physical ability of the user or an exercise result based on the received sensor data. In an embodiment, when the user exercises wearing the wearable apparatus 100, the wearable apparatus 100 may obtain sensor data including motion information of the user using sensors and transmit the obtained sensor data to the electronic device 210. The electronic device 210 may extract a motion value of the user from the sensor data and evaluate an exercise posture of the user based on the extracted motion value. The electronic device 210 may provide the user with an exercise posture measured value and exercise posture evaluation information related to the exercise posture of the user through a graphical user interface (GUI).

In an embodiment, the electronic device 210 may execute a program (e.g., an application) configured to control the wearable apparatus 100, and the user may adjust an operation or a set value of the wearable apparatus 100 (e.g., the magnitude of torque output from a driving module (e.g., the driving modules 35 and 45 of FIG. 3), the volume of audio output from a sound output module (e.g., a sound output module 550 of FIGS. 5A and 5B), or the brightness of a lighting unit (e.g., a lighting unit 85 of FIG. 3) through the corresponding program. The program executed by the electronic device 210 may provide a GUI for interaction with the user. The electronic device 210 may be a device in various forms. For example, the electronic device 210 may include a portable communication device (e.g., a smart phone), a computer device, an access point, a portable multimedia device, or a home appliance (e.g., a television, an audio device, or a projector device) but is not limited thereto.

According to an embodiment, the electronic device 210 may be connected to the server 230 using short-range wireless communication or cellular communication. The server 230 may receive user profile information of the user who uses the wearable apparatus 100 from the electronic device 210 and store and manage the received user profile information. The user profile information may include, for example, information about at least one of the name, age, gender, height, weight, or body mass index (BMI). The server 230 may receive exercise history information about an exercise performed by the user from the electronic device 210 and store and manage the received exercise history information. The server 230 may provide the electronic device 210 with various exercise programs or physical ability measurement programs that may be provided to the user.

According to an embodiment, the wearable apparatus 100 and/or the electronic device 210 may be connected to the other wearable apparatus 220. The other wearable apparatus 220 may include, for example, wireless earphones 222, a smart watch 224, or smart glasses 226 but is not limited thereto. In an embodiment, the smart watch 224 may measure a biosignal including heart rate information of the user and transmit the measured biosignal to the electronic device 210 and/or the wearable apparatus 100. The electronic device 210 may estimate the heart rate information (e.g., the current heart rate, maximum heart rate, and average heart rate) of the user based on the biosignal received from the smart watch 224 and provide the estimated heart rate information to the user.

In an embodiment, the exercise result information, physical ability information, and/or exercise posture evaluation information evaluated by the electronic device 210 may be transmitted to the other wearable apparatus 220 and provided to the user through the other wearable apparatus 220. The state information of the wearable apparatus 100 may be transmitted to the other wearable apparatus 220 and may be provided to the user through the other wearable apparatus 220. In an embodiment, the wearable apparatus 100, the electronic device 210, and the other wearable apparatus 220 may be connected to each other through wireless communication (e.g., Bluetooth communication or wireless-fidelity (Wi-Fi) communication).

In an embodiment, the wearable apparatus 100 may provide (or output) feedback (e.g., visual feedback, auditory feedback, or haptic feedback) corresponding to the state of the wearable apparatus 100 according to the control signal received from the electronic device 210. For example, the wearable apparatus 100 may provide visual feedback through the lighting unit (e.g., the lighting unit 85 of FIG. 3) and provide auditory feedback through the sound output module (e.g., the sound output module 550 of FIGS. 5A and 5B). The wearable apparatus 100 may include a haptic module and provide haptic feedback as vibration to the body of the user through the haptic module. The electronic device 210 may also provide (or output) feedback (e.g., visual feedback, auditory feedback, or haptic feedback) corresponding to the state of the wearable apparatus 100.

In an embodiment, the electronic device 210 may present a personalized exercise goal to the user in the exercise assistance mode. The personalized exercise goal may include respective target amounts of exercise for exercise types (e.g., strength exercise, balance exercise, and aerobic exercise) desired by the user and determined by the electronic device 210 and/or the server 230. When the server 230 determines a target amount of exercise, the server 230 may transmit information about the determined target amount of exercise to the electronic device 210. The electronic device 210 may personalize and present the target amounts of exercise for the exercise types, such as strength exercise, aerobic exercise, and balance exercise, according to a desired exercise program (e.g., squat, split lunge, or a lunge and knee up) and/or the physical characteristics (e.g., the age, height, weight, and BMI) of the user. The electronic device 210 may display a GUI screen displaying the target amounts of exercise for the respective exercise types on a display.

In an embodiment, the electronic device 210 and/or the server 230 may include a database in which information about a plurality of exercise programs to be provided to the user through the wearable apparatus 100 is stored. To achieve an exercise goal of the user, the electronic device 210 and/or the server 230 may recommend an exercise program suitable for the user. The exercise goal may include, for example, at least one of muscle strength improvement, physical strength improvement, cardiovascular endurance improvement, core stability improvement, flexibility improvement, or symmetry improvement. The electronic device 210 and/or the server 230 may store and manage the exercise program performed by the user, the results of performing the exercise program, and the like.

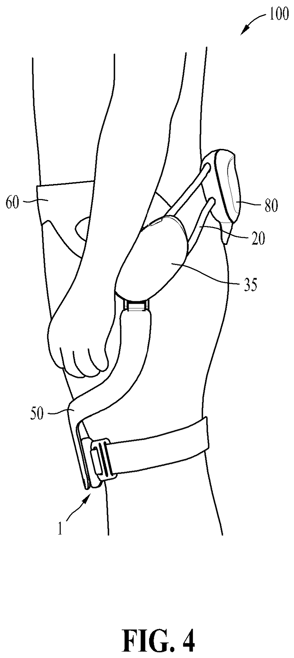

FIG. 3 is a schematic rear view of a wearable apparatus according to an embodiment. FIG. 4 is a left side view of the wearable apparatus according to an embodiment.

Referring to FIGS. 3 and 4, the wearable apparatus 100 according to an embodiment may include the base body 80, the waist support frame 20, the driving modules 35 and 45, the leg driving frames 50 and 55, thigh fastening portions 1 and 2, and a waist fastening portion 60. The base body 80 may include the lighting unit 85. In an embodiment, at least one (e.g., the lighting unit 85) of these components may be omitted from the wearable apparatus 100, or one or more other components (e.g., a haptic module) may be added to the wearable apparatus 100.

The base body 80 may be positioned on the lumbar region of a user while the user is wearing the wearable apparatus 100. The base body 80 may be mounted on the lumbar region of the user to provide a cushioning feeling to the lower back of the user and may support the lower back of the user. The base body 80 may be hung on the hip region (an area of the hips) of the user to prevent or reduce chances of the wearable apparatus 100 from being separated downward due to gravity while the user is wearing the wearable apparatus 100. The base body 80 may distribute a portion of the weight of the wearable apparatus 100 to the lower back of the user while the user is wearing the wearable apparatus 100. The base body 80 may be connected, directly or indirectly, to the waist support frame 20. Waist support frame-connecting elements (not shown) to be connected, directly or indirectly, to the waist support frame 20 may be provided at both end portions of the base body 80.

In an embodiment, the lighting unit 85 may be arranged on the outer side of the base body 80. The lighting unit 85 may include one or more lighting sources (e.g., a light emitting diode (LED)). The lighting unit 85 may emit light under the control by a control module (not shown) (e.g., the control module 510 of FIGS. 5A and 5B, comprising processing circuitry). In some embodiments, the control module may control the lighting unit 85 to provide (or output) visual feedback corresponding to the state of the wearable apparatus 100 to the user through the lighting unit 85.

The waist support frame 20 may extend from both end portions of the base body 80. The lumbar region of the user may be accommodated inside the waist support frame 20. The waist support frame 20 may include at least one rigid body beam. Each beam may be in a curved shape having a preset curvature to enclose the lumbar region of the user. The waist fastening portion 60 may be connected to an end portion of the waist support frame 20. The driving modules 35 and 45 may be connected to the waist support frame 20.

In an embodiment, the control module, an IMU (not shown) (e.g., the IMU 135 of FIG. 1 or an IMU 522 of FIG. 5B), a communication module (not shown) (e.g., a communication module 516 of FIGS. 5A and 5B, comprising communication circuitry), and a battery (not shown) may be arranged inside the base body 80. The base body 80 may protect the control module, the IMU, the communication module, and the battery. The control module may generate a control signal for controlling the operation of the wearable apparatus 100. The control module may include a control circuit including a processor configured to control actuators of the driving modules 35 and 45 and a memory. The control module may further include a power supply module (not shown) configured to supply power from the battery to each of the components of the wearable apparatus 100.

In an embodiment, the wearable apparatus 100 may include a sensor module (not shown) (e.g., the sensor module 520 of FIG. 5A) configured to obtain sensor data from at least one sensor. The sensor module may obtain sensor data that changes according to the motion of the user. In an embodiment, the sensor module may obtain sensor data including motion information of the user and/or motion information of the components of the wearable apparatus 100. The sensor module may include, for example, an IMU (e.g., the IMU 135 of FIG. 1 or the IMU 522 of FIG. 5B) configured to measure an upper body motion value of the user or a motion value of the waist support frame 20 and an angle sensor (e.g., the angle sensor 125 of FIG. 1 or a first angle sensor 524 and a second angle sensor 524-1 of FIG. 5B) configured to measure a hip joint angle value of the user or a motion value of the leg support frames 50 and 55 but is not limited thereto. For example, the sensor module may further include at least one of a position sensor, a temperature sensor, a biosignal sensor, or a proximity sensor.

The waist fastening portion 60 may be connected to the waist support frame 20 to fasten the waist support frame 20 to the waist of the user. The waist fastening portion 60 may include, for example, a pair of belts.

The driving modules 35 and 45 may generate an external force (or torque) to be applied to the body of the user based on the control signal generated by the control module. For example, the driving modules 35 and 45 may generate an assistance force or resistance force to be applied to the legs of the user. In an embodiment, the driving modules 35 and 45 may include a first driving module 45 and a second driving module 35, wherein the first driving module 45 is disposed in a position corresponding to the position of the right hip joint of the user and a second driving module 35 is disposed in a position corresponding to the position of the left hip joint of the user. The first driving module 45 may include a first actuator and a first joint member, and the second driving module 35 may include a second actuator and a second joint member. The first actuator may provide power to be transmitted to the first joint member, and the second actuator may provide power to be transmitted to the second joint member. The first actuator and the second actuator may each include a motor configured to generate power (or a torque) by receiving electric power from the battery. When the motor is supplied with electric power and driven, the motor may generate a force (an assistance force) for assisting the body motion of the user or a force (a resistance force) for hindering the body motion of the user. For example, the motor may be a three-phase motor. In an embodiment, the control module may adjust the strength and direction of the force generated by the motor by adjusting the voltage and/or current supplied to the motor.

In an embodiment, the first joint member and the second joint member may receive power from the first actuator and the second actuator, respectively, and may apply an external force to the body of the user based on the received power. The first joint member and the second joint member may be arranged at positions corresponding to the joints of the user, respectively. One side of the first joint member may be connected to the first actuator, and the other side of the first joint member may be connected to a first leg support frame 55. The first joint member may be rotated by the power received from the first actuator. An encoder or a Hall sensor that may operate as an angle sensor configured to measure the rotational angle of the first joint member (corresponding to the joint angle of the user) may be arranged on one side of the first joint member. One side of the second joint member may be connected to the second actuator, and the other side of the second joint member may be connected to a second leg support frame 50. The second joint member may be rotated by the power received from the second actuator. An encoder or a Hall sensor that may operate as an angle sensor configured to measure the rotational angle of the second joint member may be arranged on one side of the second joint member.

In an embodiment, the first actuator may be arranged in a lateral direction of the first joint member, and the second actuator may be arranged in a lateral direction of the second joint member. A rotation axis of the first actuator and a rotation axis of the first joint member may be spaced apart from each other, and a rotation axis of the second actuator and a rotation axis of the second joint member may also be spaced apart from each other. However, embodiments are not limited thereto, and an actuator and a joint member may share a rotation axis. In an embodiment, each actuator may be spaced apart from a corresponding joint member. In this case, the driving modules 35 and 45 may further include a power transmission module (not shown) configured to transmit power from the actuator to the joint member. The power transmission module may be a rotary body, such as a gear, or a longitudinal member, such as a wire, a cable, a string, a spring, a belt, or a chain. However, the scope of the embodiment is not limited by the positional relationship between an actuator and a joint member and the power transmission structure described above.

In an embodiment, the leg support frames 50 and 55 may support a leg (e.g., the thigh) of the user when the wearable apparatus 100 is worn on the leg of the user. For example, the leg support frames 50 and 55 may transmit power (a torque) generated by the driving modules 35 and 45 to the thighs of the user, and the power may act as an external force to be applied to the motion of the legs of the user. As end portions of the leg support frames 50 and 55 may be connected to the joint member to rotate and the other end portions of the leg support frames 50 and 55 may be connected to the thigh fastening portions 1 and 2, the leg support frames 50 and 55 may transmit the power generated by the driving modules 35 and 45 to the thighs of the user while supporting the thighs of the user. For example, the leg support frames 50 and 55 may push or pull the thighs of the user. The leg support frames 50 and 55 may extend in the longitudinal direction of the thighs of the user. The leg support frames 50 and 55 may be bent to surround at least a portion of the circumference of the thighs of the user. The leg support frames 50 and 55 may include the first leg support frame 55 configured to support the right leg of the user and the second leg support frame 50 configured to support the left leg of the user.

The thigh fastening portions 1 and 2 may be connected to the leg support frames 50 and 55 and may fasten the leg support frames 50 and 55 to the thighs. The thigh fastening portions 1 and 2 may include the first thigh fastening portion 2 configured to fasten the first leg support frame 55 to the right thigh of the user and the second thigh fastening portion 1 configured to fasten the second leg support frame 50 to the left thigh of the user.

In an embodiment, the first thigh fastening portion 2 may include a first cover, a first fastening frame, and a first strap, and the second thigh fastening portion 1 may include a second cover, a second fastening frame, and a second strap. The first cover and the second cover may apply torques generated by the driving modules 35 and 45 to the thighs of the user. The first cover and the second cover may be arranged on one side of the thighs of the user to push or pull the thighs of the user. The first cover and the second cover may be arranged on the front surfaces of the thighs of the user. The first cover and the second cover may be arranged in the circumferential directions of the thighs of the user. The first cover and the second cover may extend to both sides from the other end portions of the leg support frames 50 and 55 and may include curved surfaces corresponding to the thighs of the user. Ends of the first cover and the second cover may be connected to the fastening frames, and the other ends of the first cover and the second cover may be connected to the straps.

The first fastening frame and the second fastening frame may be arranged, for example, to surround at least some portions of the circumferences of the thighs of the user, thereby preventing or reducing chances of the thighs of the user from being separated from the leg support frames 50 and 55. The first fastening frame may have a J fastening structure that connects the first cover to the first strap, and the second fastening frame may have a fastening structure that connects the second cover to the second strap.

The first strap may enclose the remaining portion of the circumference of the right thigh of the user that is not covered by the first cover and the first fastening frame, and the second strap may enclose the remaining portion of the circumference of the left thigh of the user that is not covered by the second cover and the second fastening frame. The first strap and the second strap may include, for example, an elastic material (e.g., a band).

FIGS. 5A and 5B are diagrams illustrating a configuration of a control system of a wearable apparatus, according to an embodiment.

Referring to FIG. 5A, a wearable apparatus 500 (e.g., the wearable apparatus 100) may include a control module 510, a communication module 516, a sensor module 520, a driving module 530, an input module 540, and a sound output module 550. In an embodiment, at least one (e.g., the sound output module 550) of the components described above may be omitted from the wearable apparatus 500, or one or more other components (e.g., a haptic module) may be added to the wearable apparatus 500.

The driving module 530 may include a motor 534 configured to generate power (e.g., a torque) and a motor driver circuit 532 configured to drive the motor 534. In the example of FIG. 5A, the driving module 530 including one motor driver circuit 532 and one motor 534 is illustrated, however this is only an example. Referring to FIG. 5B, a wearable apparatus 500-1 may include a plurality of (e.g., two or more) motor driver circuits 532 and 532-1 and a plurality of (e.g., two or more) motors 534 and 534-1. The driving module 530 including the motor driver circuit 532 and the motor 534 may correspond to the first driving module 45 of FIG. 3, and a driving module 530-1 including the motor driver circuit 532-1 and the motor 534-1 may correspond to the second driving module 35 of FIG. 3. The following descriptions of the motor driver circuit 532 and the motor 534 may also be respectively applicable to the motor driver circuit 532-1 and the motor 534-1 illustrated in FIG. 5B.

Referring back to FIG. 5A, the sensor module 520 may include a sensor circuit including at least one sensor. The sensor module 520 may obtain sensor data including motion information of a user or motion information of the wearable apparatus 500. The sensor module 520 may transmit the obtained sensor data to the control module 510. The sensor module 520 may include an IMU 522 and an angle sensor (e.g., a first angle sensor 524 and a second angle sensor 524-1) as illustrated in FIG. 5B. The IMU 522 may measure an upper body motion value of the user. For example, the IMU 522 may sense X-axis, Y-axis, and Z-axis accelerations and X-axis, Y-axis, and Z-axis angular velocities according to the motion of the user. The IMU 522 may be used to measure, for example, at least one of a forward and backward tilt, a left and right tilt, or a rotation of the body of the user. In addition, the IMU 522 may obtain motion values (e.g., acceleration values and angular velocity values) of a waist support frame (e.g., the waist support frame 20 of FIG. 3) of the wearable apparatus 500. The motion values of the waist support frame may correspond to upper body motion values of the user.

The angle sensor may measure a hip joint angle value according to a leg motion of the user. Sensor data that may be measured by the angle sensor may include, for example, a hip joint angle value of the right leg, a hip joint angle value of the left leg, and information on a motion direction of a leg. For example, the first angle sensor 524 of FIG. 5B may obtain the hip joint angle value of the right leg of the user, and the second angle sensor 524-1 may obtain the hip joint angle value of the left leg of the user. The first angle sensor 524 and the second angle sensor 524-1 may each include, for example, an encoder and/or a Hall sensor. Furthermore, the angle sensors may obtain motion values of the leg support frames of the wearable apparatus 500. For example, the first angle sensor 524 may obtain a motion value of the first leg support frame 55, and the second angle sensor 524-1 may obtain a motion value of the second leg support frame 50. The motion values of the leg support frames may correspond to the hip joint angle values.

In an embodiment, the sensor module 520 may further include at least one of a position sensor configured to obtain a position value of the wearable apparatus 500, a proximity sensor configured to sense the proximity of an object, a biosignal sensor configured to detect a biosignal of the user, or a temperature sensor configured to measure an ambient temperature.

The input module 540 may receive a command or data to be used by a component (e.g., a processor 512) of the wearable apparatus 500 from the outside (e.g., the user) of the wearable apparatus 500. The input module 540 may include an input component circuit. The input module 540 may include, for example, a key (e.g., a button) or a touch screen.

The sound output module 550 may output a sound signal to the outside of the wearable apparatus 500. The sound output module 550 may provide auditory feedback to the user. For example, the sound output module 550 may include a speaker configured to play back a guiding sound signal (e.g., an operation start sound, an operation error alarm, or an exercise start alarm), music content, or a guiding voice for auditorily informing predetermined information (e.g., exercise result information or exercise posture evaluation information).

In an embodiment, the wearable apparatus 500 may further include a battery (not shown) to supply power to each component of the wearable apparatus 500. The wearable apparatus 500 may convert the power of the battery into power suitable for an operating voltage of each component of the wearable apparatus 500 and supply the converted power to each component.

The driving module 530 may generate an external force to be applied to the leg of the user under the control by the control module 510. The driving module 530 may generate a torque to be applied to the legs of the user based on a control signal generated by the control module 510. The control module 510 may transmit the control signal to the motor driver circuit 532. The motor driver circuit 532 may control the operation of the motor 534 by generating a current signal (or voltage signal) corresponding to the control signal and supplying the generated current signal to the motor 534. In some cases, the current signal may not be supplied to the motor 534. When the motor 534 is supplied with the current signal and driven, the motor 534 may generate a torque for an assistance force for assisting the leg motion of the user or a resistance force for hindering the leg motion of the user.

The control module 510 may control the overall operation of the wearable apparatus 500 and may generate a control signal to control each component (e.g., the communication module 516 and the driving module 530). The control module 510 may include the processor 512 and a memory 514.

The processor 512 may execute, for example, software to control at least one other component (e.g., a hardware or software component) of the wearable apparatus 500 connected to the processor 512 and may perform a variety of data processing or operations. The software may include an application for providing a GUI. According to an embodiment, as at least a portion of the data processing or operations, the processor 512 may store, in the memory 514, instructions or data received from another component (e.g., the communication module 516), process the instructions or data stored in the memory 514, and store result data after processing in the memory 514. According to an embodiment, the processor 512 may include a main processor (e.g., a central processing unit (CPU) or an application processor (AP)) or an auxiliary processor (e.g., a graphics processing unit (GPU), a neural processing unit (NPU), an image signal processor (ISP), a sensor hub processor, or a communication processor (CP)) that is operable independently of or in conjunction with the main processor. The auxiliary processor may be implemented separately from the main processor or as part of the main processor.

Each "processor" herein includes processing circuitry, and/or may include multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions.

The memory 514 may store various pieces of data used by at least one component (e.g., the processor 512) of the control module 510. The various pieces of data may include, for example, software, sensor data, and input data or output data for instructions related thereto. The memory 514 may include a volatile memory or a non-volatile memory (e.g., random-access memory (RAM), dynamic RAM (DRAM), or static RAM (SRAM)).

The communication module 516 may support establishing a direct (e.g., wired) communication channel or a wireless communication channel between the control module 510 and another component of the wearable apparatus 500 or an external electronic device (e.g., the electronic device 210 or the other wearable apparatus 220 of FIG. 2) and performing communication via the established communication channel. The communication module 516 may include a communication circuit configured to perform a communication function. For example, the communication module 516 may receive a control signal from an electronic device (e.g., the electronic device 210) and transmit the sensor data obtained by the sensor module 520 to the electronic device. According to an embodiment, the communication module 516 may include one or more CPs (not shown) that are operable independently of the processor 512 and that support a direct (e.g., wired) communication or a wireless communication. According to an embodiment, the communication module 516 may include a wireless communication module (e.g., a cellular communication module, a short-range wireless communication module, or a global navigation satellite system (GNSS) communication module), and/or a wired communication module. A corresponding one of these communication modules may communicate with another component of the wearable apparatus 500 and/or an external electronic device via a short-range communication network, such as Bluetooth, Wi-Fi, or infrared data association (IrDA) or a long-range communication network, such as a legacy cellular network, a fifth generation (5G) network, a next-generation communication network, the Internet, or a computer network (e.g., a local area network (LAN) or a wide area network (WAN)).

In an embodiment, each of the wearable apparatuses 500 and 500-1 may further include a haptic module (not shown). The haptic module may provide haptic feedback to the user under the control by the processor 512. The haptic module may convert an electrical signal into a mechanical stimulus (e.g., a vibration or a movement) or an electrical stimulus which may be recognized by a user via their tactile sensation or kinesthetic sensation. The haptic module may include a motor, a piezoelectric element, or an electrical stimulation device. In an embodiment, the haptic module may be positioned in at least one of the base body (e.g., the base body 80), the first thigh fastening portion 2, or the second thigh fastening portion 1.

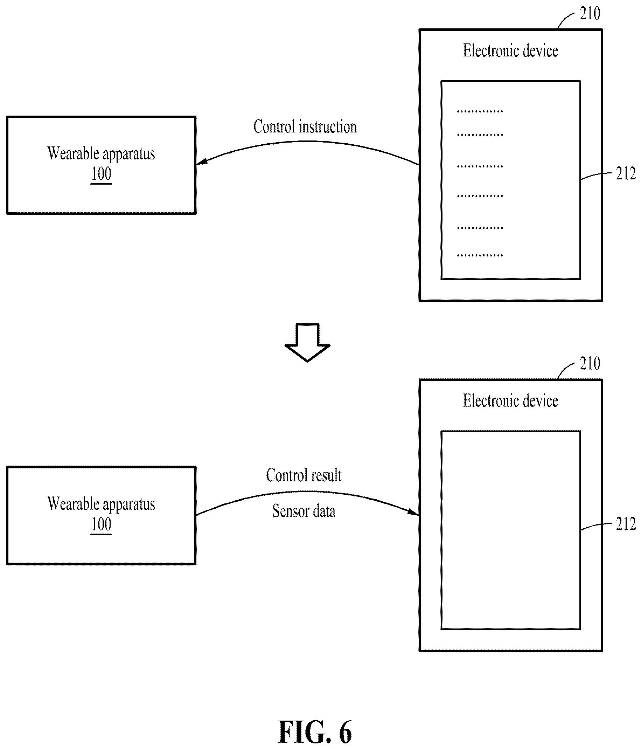

FIG. 6 is a diagram illustrating an interaction between a wearable apparatus and an electronic device, according to an embodiment.

Referring to FIG. 6, the wearable apparatus 100 may communicate with the electronic device 210. For example, the electronic device 210 may be a user terminal of a user who uses the wearable apparatus 100 or a controller device dedicated to the wearable apparatus 100. According to an embodiment, the wearable apparatus 100 and the electronic device 210 may be connected to each other through short-range wireless communication (e.g., Bluetooth communication or Wi-Fi communication).

In an embodiment, the electronic device 210 may check a state of the wearable apparatus 100 or execute an application to control or operate the wearable apparatus 100. A screen of a UI may be displayed to control an operation of the wearable apparatus 100 or determine an operation mode of the wearable apparatus 100 on a display 212 of the electronic device 210 through the execution of the application. The UI may be, for example, a GUI.

In an embodiment, the user may input an instruction for controlling the operation of the wearable apparatus 100 (e.g., an execution instruction to a walking assistance mode, an exercise assistance mode, or a physical ability measurement mode) or change settings of the wearable apparatus 100 through a GUI screen on the display 212 of the electronic device 210. The electronic device 210 may generate a control instruction (or control signal) corresponding to an operation control instruction or a setting change instruction input by the user and transmit the generated control instruction to the wearable apparatus 100. The wearable apparatus 100 may operate according to the received control instruction and transmit a control result according to the control instruction and/or sensor data measured by the sensor module of the wearable apparatus 100 to the electronic device 210. The electronic device 210 may provide the user with result information (e.g., walking ability information, exercise ability information, or exercise posture evaluation information) derived by analyzing the control result and/or the sensor data through the GUI screen.

In an embodiment, the wearable apparatus 100 may receive a diagnostic request from the electronic device 210. The wearable apparatus 100 may perform a diagnostic operation (e.g., an operation of diagnosing a failure in a discharging circuit to be described below) according to the diagnostic request received from the electronic device 210.

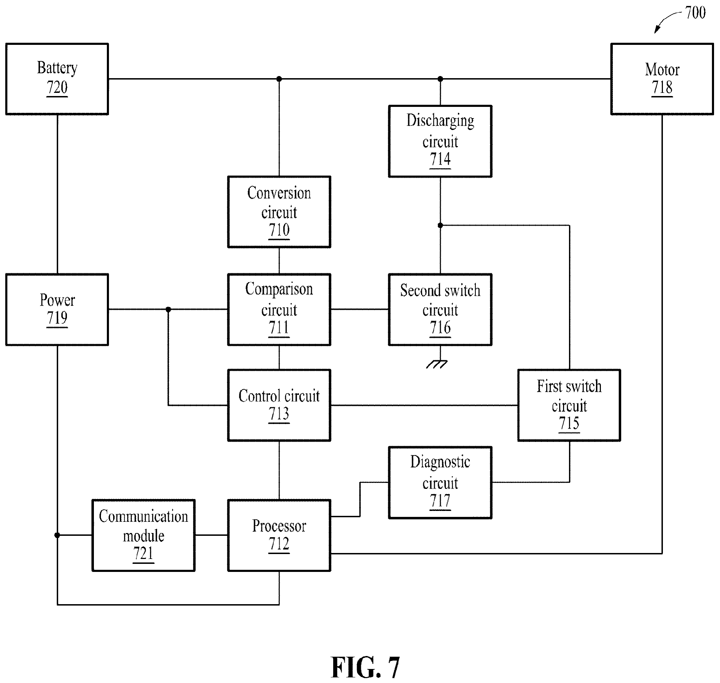

FIG. 7 is a block diagram illustrating an example of a configuration of a wearable apparatus, according to an embodiment.

Referring to FIG. 7, a wearable apparatus 700 (e.g., the wearable apparatus 100, the wearable apparatus 500, and the wearable apparatus 500-1) according to an embodiment may include a conversion circuit 710, a comparison circuit 711, a processor 712 (e.g., the processor 512), a control circuit 713, a discharging circuit 714, a first switch circuit 715, a second switch circuit 716, a diagnostic circuit 717, a motor 718, a power 719, a battery 720, and a communication module 721.

According to an embodiment, the conversion circuit 710, the comparison circuit 711, the control circuit 713, the discharging circuit 714, the first switch circuit 715, the second switch circuit 716, and the diagnostic circuit 717 may be circuits for the motor 718. The motor 718 of the wearable apparatus 700 may be a motor for providing an external force to one leg. The wearable apparatus 700 may further include another motor for providing an external force to the other leg and may include a conversion circuit, a comparison circuit, a control circuit, a discharging circuit, a first switch circuit, a second switch circuit, and a diagnostic circuit for the other motor. The descriptions of the conversion circuit 710, the comparison circuit 711, the control circuit 713, the discharging circuit 714, the first switch circuit 715, the second switch circuit 716, and the diagnostic circuit 717 may respectively apply to the descriptions of the conversion circuit, the comparison circuit, the control circuit, the discharging circuit, the first switch circuit, the second switch circuit, and the diagnostic circuit for the other motor.

According to an embodiment, at least some of the conversion circuit 710, the comparison circuit 711, the control circuit 713, the first switch circuit 715, the second switch circuit 716, or the diagnostic circuit 717 or all of them may be implemented as a diagnostic module.

According to an embodiment, at least some of the conversion circuit 710, the comparison circuit 711, the processor 712, the control circuit 713, the first switch circuit 715, the second switch circuit 716, the diagnostic circuit 717, or the communication module 721 or all of them may be located on a printed circuit board (PCB). Some components (e.g., at least some of the conversion circuit 710, the comparison circuit 711, the processor 712, the control circuit 713, the first switch circuit 715, the second switch circuit 716, the diagnostic circuit 717, or the communication module 721 or all of them) of the wearable apparatus 700 may be located on the PCB, which may represent a first printed board assembly (PBA) (or a main PBA). As described below, heat may be generated by the discharging circuit 714, and to ensure that the heat generated by the discharging circuit 714 has minimal impact on the first PBA, the discharging circuit 714 may be separated from (or spaced a predetermined distance apart from) the first PBA.

According to an embodiment, the power 719 may generate a static power source VCC required by the wearable apparatus 700. For example, the power 719 may provide VCC to the comparison circuit 711, the control circuit 713, and the like.

According to an embodiment, when a user of the wearable apparatus 700 moves, the motor 718 may be moved (or rotated) by the movement of the user. The movement (or rotation) of the motor 718 may generate an electromotive force (hereinafter, referred to as "motor electromotive force") by the motor 718.

According to an embodiment, the conversion circuit 710 may generate a voltage VDC based on the voltage VMOT (or the voltage input to the motor 718) of the power used by the motor 718.

The voltage VMOT may be, for example, the voltage of the motor electromotive force. For example, in the resistance mode of the wearable apparatus 700, the motor 718 may use the motor electromotive force to generate the external force (or torque) to be provided to the user. In the exercising state, as described below, the motor 718 may generate the external force (or torque) to be provided to the user by receiving the motor electromotive force.

The voltage VMOT may be, for example, the voltage of the battery 720. For example, as described below, in the pre-exercise state (or post-exercise state), the wearable apparatus 700 may supply the power of the battery 720 to the motor 718 to determine whether the discharging circuit 714 is in a normal state.

According to an embodiment, the conversion circuit 710 may include a plurality of resistors and may generate the voltage VDC by converting the voltage VMOT to the voltage VDC through voltage division using the plurality of resistors. According to an embodiment, the conversion circuit 710 may include an operational amplifier (OP AMP) and may generate the voltage VDC by converting the voltage VMOT to the voltage VDC through the OP AMP.

According to an embodiment, the comparison circuit 711 may compare the voltage VDC received from the conversion circuit 710 with a reference voltage VREF. The comparison circuit 711 may output a first signal (e.g., a voltage signal) when the voltage VDC is equal to or greater than the reference voltage VREF. A state in which the voltage VDC is equal to or greater than the reference voltage VREF may be an overvoltage state of the motor 718 or a state in which an excessive motor electromotive force is generated by the motor 718. The comparison circuit 711 may not output the first signal when the voltage VDC is less than the reference voltage VREF.

According to an embodiment, the second switch circuit 716 may ensure that the motor electromotive force is transmitted to the discharging circuit 714 when receiving the first signal from the comparison circuit 711.

According to an embodiment, the discharging circuit 714 may include one or more resistors and may consume the motor electromotive force through the one or more resistors. The voltage of the motor electromotive force may be higher than the voltage of the battery 720, which may damage a component (e.g., the processor 712) of the wearable apparatus 700. To prevent or reduce such damage, the discharging circuit 714 may consume the motor electromotive force through one or more resistors when the voltage of the motor electromotive force is greater than or equal to a predetermined level.

According to an embodiment, when the motor 718 is in the overvoltage state, the processor 712 may receive the voltage VDC from the conversion circuit 710. The processor 712 may determine whether the discharging circuit 714 is in an abnormal state based on a change (or a change pattern) in the received voltage VDC. For example, when identifying that the voltage VDC increases during a predetermined time interval (e.g., a time interval from the time point when the voltage VDC is greater than or equal to the reference voltage VREF to the time point when △t elapses), the processor 712 may determine that the discharging circuit 714 is in a first abnormal state (e.g., an electrical open state of a resistor of the discharging circuit 714 and/or a state in which a wire harness of the discharging circuit 714 is disconnected). When identifying that the voltage VDC decreases beyond a predetermined level during a predetermined time interval, the processor 712 may determine that the discharging circuit 714 is in a second abnormal state (e.g., an electrical short state of the resistor of the discharging circuit 714 and/or a state in which the wire harness of the discharging circuit 714 is shorted). When identifying that the voltage VDC decreases to a predetermined level during a predetermined time interval, the processor 712 may determine that the discharging circuit 714 is in a normal state.

According to an embodiment, in an overvoltage state, a current sensor (not shown) may sense the current in the discharging circuit 714, obtain a current value, and transmit the obtained current value to the processor 712. The processor 712 may determine whether the discharging circuit 714 is in an abnormal state based on the received current value. For example, when the received current value corresponds to a predetermined current value (or a normal current value), the processor 712 may determine that the discharging circuit 714 is in a normal state. When the received current value is 0, the processor 712 may determine that the discharging circuit 714 is in the first abnormal state (e.g., an electrical open state of the resistor of the discharging circuit 714 and/or a state in which the wire harness of the discharging circuit 714 is shorted). When the received current value is higher than a predetermined current value (or normal current value) by a predetermined level, the processor 712 may determine that the discharging circuit 714 is in a second abnormal state (e.g., an electrical short state of the resistor of the discharging circuit 714 and/or a state in which the wire harness of the discharging circuit 714 is shorted).

According to an embodiment, the processor 712 may determine whether the discharging circuit 714 is in an abnormal state based on the voltage V_DIAF of the diagnostic circuit 717 when the processor 712 is not in an overvoltage state (e.g., when the voltage VDC is less than the reference voltage VREF).

According to an embodiment, when not in the overvoltage state, the processor 712 may generate a diagnostic request signal DIAG_CON for the discharging circuit 714. For example, the processor 712 may receive a diagnostic request for the discharging circuit 714 from the electronic device 210 via the communication module 721 and generate the diagnostic request signal DIAG_CON based on the received diagnostic request. In another example, the processor 712 may periodically generate the diagnostic request signal DIAG_CON to periodically monitor the discharging circuit 714 for any abnormalities.

According to an embodiment, when not in the overvoltage state, the processor 712 may control the first switch circuit 715 to allow the voltage of the battery 720 to be transmitted to the discharging circuit 714.

According to an embodiment, the control circuit 713 may output a second signal (e.g., a voltage V_FDIAG with a low value) to control the first switch circuit 715 based on the diagnostic request signal.

According to an embodiment, the first switch circuit 715 may electrically connect the discharging circuit 714 to the diagnostic circuit 717 when receiving the second signal (e.g., the voltage V_FDIAG with a low value) from the control circuit 713.

According to an embodiment, the diagnostic circuit 717 may include one or more electrical components (e.g., resistors, etc.). When the diagnostic circuit 717 is electrically connected to the discharging circuit 714 through the first switch circuit 715, the diagnostic circuit 717 may generate a diagnostic voltage V_DIAG based on the voltage of the motor electromotive force VMOT, one or more resistors within the discharging circuit 714, and one or more electrical components within the diagnostic circuit 717. The diagnostic circuit 717 may transmit the diagnostic voltage V_DIAG to the processor 712.

According to an embodiment, the processor 712 may determine whether the discharging circuit 714 is in an abnormal state based on the voltage value of the diagnostic voltage V_DIAG received from the diagnostic circuit 717. For example, when the voltage value of the diagnostic voltage V_DIAG corresponds to a predetermined voltage value (or normal voltage value), the processor 712 may determine that the discharging circuit 714 is in a normal state. When the voltage value of the diagnostic voltage V_DIAG is 0, the processor 712 may determine that the discharging circuit 714 is in the first abnormal state (e.g., the electrical open state of the resistor of the discharging circuit 714 and/or the state in which the wire harness of the discharging circuit 714 is disconnected). When the voltage value of the diagnostic voltage V_DIAG is higher than the predetermined voltage value (or normal voltage value), the processor 712 may determine that the discharging circuit 714 is in the second abnormal state (e.g., the electrical short state of the resistor of the discharging circuit 714 and/or the state in which the wire harness of the discharging circuit 714 is shorted).

According to an embodiment, when determining that the discharging circuit 714 is in an abnormal state (e.g., the first abnormal state or the second abnormal state), the processor 712 may control the communication module 721 to transmit a report indicating that the discharging circuit 714 is in the abnormal state to at least one of a cloud server or the electronic device 210.

According to an embodiment, when determining that the discharging circuit 714 is in an abnormal state while the wearable apparatus 700 provides an external force (or torque) to the user, the processor 712 may control the motor 718 to stop providing the external force (or torque) to the user. The processor 712 may control the motor 718 to halt its operation.

According to an embodiment, when the motor 718 is in an overvoltage state, the processor 712 may control the lighting unit 85 including one or more LEDs to use the motor electromotive force as a power source. The lighting unit 85 may output light corresponding to the state (e.g., abnormal state, etc.) of the wearable apparatus 700.

According to an embodiment, when the motor 718 is in an overvoltage state, the processor 712 may provide a heating function to the user based on the heat generated by the discharging circuit 714 consuming the motor electromotive force. For example, due to cold weather, the temperature of the wearable apparatus 700 may be less than or equal to a predetermined temperature. Additionally, the motor 718 may be in an overvoltage state. The processor 712 may provide the heating function to the user through the heat generated by the discharging circuit 714 consuming the motor electromotive force.

According to an embodiment, the wearable apparatus 700 may charge the battery 720 based on the motor electromotive force. For example, the wearable apparatus 700 may include a rectifier (or a rectifying circuit) and convert the motor electromotive force into power suitable for charging the battery 720. When the voltage of the motor electromotive force exceeds the allowable charging voltage of the battery 720, the wearable apparatus 700 may consume the motor electromotive force through the discharging circuit 714 and/or use the motor electromotive force as a power source for the lighting unit 85.

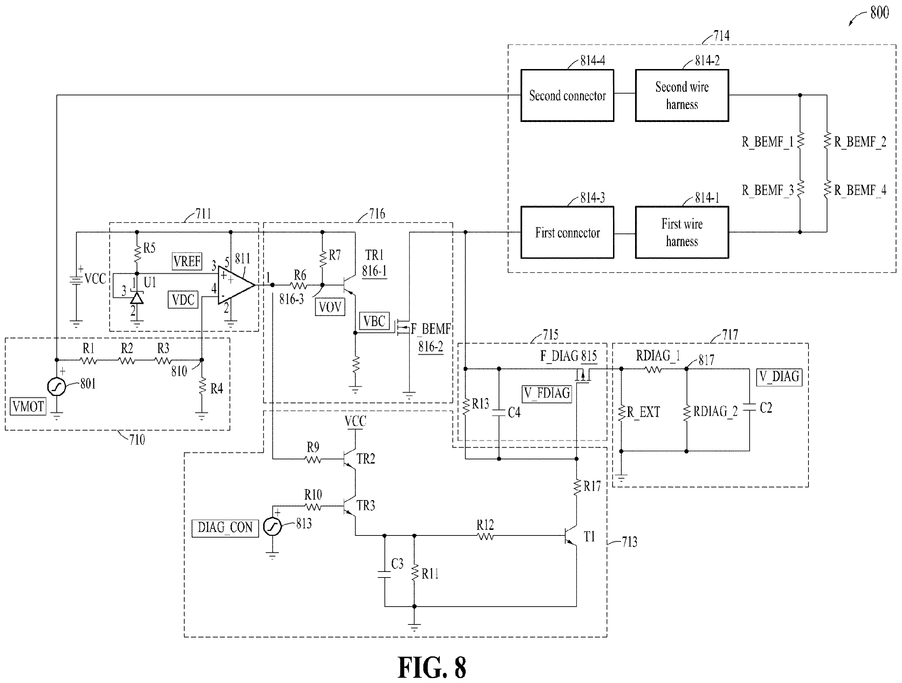

FIG. 8 is a diagram illustrating an example of a conversion circuit, a comparison circuit, a control circuit, a discharging circuit, a diagnostic circuit, a first switch circuit, and a second switch circuit of a wearable apparatus, according to an embodiment.

A circuit 800 of FIG. 8 may include the conversion circuit 710, the comparison circuit 711, the control circuit 713, the discharging circuit 714, the diagnostic circuit 717, the first switch circuit 715, and the second switch circuit 716.

A voltage VMOT 801 shown in FIG. 8 may be the voltage (or the voltage input to the motor 718) (e.g., the voltage of the motor electromotive force or the voltage of the battery 720) of the power used by the motor 718.

In the example illustrated in FIG. 8, the conversion circuit 710 may include a plurality of resistors R1, R2, R3, and R4. In the example illustrated in FIG. 8, the conversion circuit 710 may also be referred to as a voltage dividing circuit.

According to an embodiment, the conversion circuit 710 may convert a voltage VMOT to a voltage VDC that the processor 712 may receive through voltage division using the resistors R1, R2, R3, and R4. When receiving the voltage VMOT, the conversion circuit 710 may perform voltage division using the resistors R1, R2, R3, and R4 to output the voltage VDC. The voltage VDC is the voltage of a node 810 and may be expressed by Equation 1 below.

[Equation 1]

$$VDC = \frac{VMOT \cdot R4}{R1 + R2 + R3 + R4}$$

According to an embodiment, unlike the example illustrated in FIG. 8, the conversion circuit 710 may include an OP AMP and convert the voltage VMOT to the voltage VDC through the OP AMP.

In an embodiment, the processor 712 may receive the voltage VDC from the conversion circuit 710. As described in detail below, the processor 712 may determine whether the discharging circuit 714 is in an abnormal state based on the change in the voltage VDC.

According to an embodiment, the comparison circuit 711 may include a comparator 811. The comparator 811 may compare the voltage VDC with a reference voltage VREF. When the voltage VDC is greater than or equal to the reference voltage VREF, the comparator 811 may output a first signal (e.g., a voltage signal) to the second switch circuit 716.

According to an embodiment, the second switch circuit 716 may include a transistor TR1 816-1 and a transistor F_BEMF 816-2. When the second switch circuit 716 receives the first signal from the comparison circuit 711, the voltage VOV at a node 816-3 may have a low value (e.g., 4.2 volts (V)) due to resistors R6 and R7 within the second switch circuit 716. When the second switch circuit 716 does not receive the first signal from the comparison circuit 711, the voltage VOV at the node 816-3 may have a high value (e.g., 5 V).

According to an embodiment, when the high-value voltage VOV is applied to the gate of the transistor TR1 816-1, the transistor TR1 816-1 may output a voltage. When the transistor TR1 816-1 outputs the voltage, a voltage VBC may have a high value (e.g., 5 V). When the high-value voltage VBC is applied to the gate of the transistor F_BEMF 816-2, the transistor F_BEMF 816-2 may be turned on. The second switch circuit 716 may be in a turned-on state. The high-value voltage VBC may correspond to a signal (or control signal) that may turn on the transistor F_BEMF 816-2. As the transistor F_BEMF 816-2 is turned on, a motor electromotive force may be transmitted to the discharging circuit 714.

According to an embodiment, the discharging circuit 714 may include a plurality of resistors R_BEMF_1, R_BEMF_2, R_BEMF_3, and R_BEMF_4, a plurality of wire harnesses 814-1 and 814-2, and a plurality of connectors 814-3 and 814-4. Depending on the implementation, the discharging circuit 714 may refer only to a circuit including the resistors R_BEMF_1, R_BEMF_2, R_BEMF_3, and R_BEMF_4. In this case, the discharging circuit 714 may be connected to the first PBA (or main PBA) (e.g., a PBA including the processor 712) via the wire harnesses 814-1 and 814-2 and the connectors 814-3 and 814-4. The conversion circuit 710, the comparison circuit 711, the processor 712, the control circuit 713, the diagnostic circuit 717, the first switch circuit 715, the second switch circuit 716, the power 719, and the communication module 721 comprising communication circuitry may be located on a PCB. The connectors 814-3 and 814-4 may allow the discharging circuit 714 to be connected to or disconnected from the PCB. Depending on the implementation, the connectors 814-3 and 814-4 may be omitted. When the connectors 814-3 and 814-4 are omitted, the discharging circuit 714 may be connected to the PCB by soldering.

According to an embodiment, the discharging circuit 714 may consume the motor electromotive force through the plurality of resistors R_BEMF_1, R_BEMF_2, R_BEMF_3, and R_BEMF_4. In the example illustrated in FIG. 8, the discharging circuit 714 may include four resistors R_BEMF_1, R_BEMF_2, R_BEMF_3, and R_BEMF_4, but this is only an example.

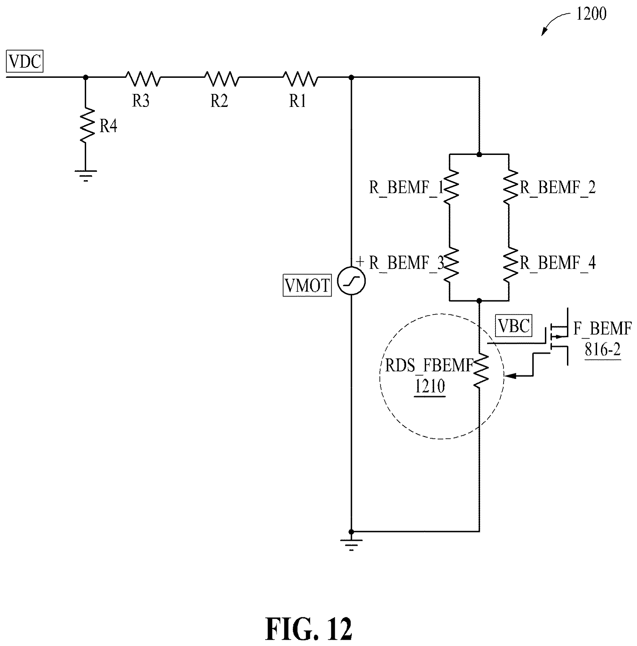

According to an embodiment, in an overvoltage state (or a state in which the discharging circuit 714 consumes the motor electromotive force), the processor 712 may determine whether the discharging circuit 714 is in an abnormal state based on at least one of the amount of change in the voltage VDC or the current value of the discharging circuit 714. This is described below with reference to FIGS. 12, 13, and 14.

According to an embodiment, when not in an overvoltage state, the processor 712 may generate a diagnostic request signal DIAG_CON for the discharging circuit 714. In the example illustrated in FIG. 8, a voltage source 813 may represent the diagnostic request signal DIAG_CON.

According to an embodiment, when receiving the diagnostic request signal DIAG_CON from the processor 712, the control circuit 713 may output a second signal (e.g., a voltage V_FDIAG with a low value) that causes the first switch circuit 715 to be in a turned-on state.

According to an embodiment, the first switch circuit 715 may include a transistor F_DIAG 815. The second signal (e.g., the voltage V_FDIAG with a low value) from the control circuit 713 may be applied to the gate of the transistor F_DIAG 815. As a result, the transistor F_DIAG 815 may be turned on. In other words, the first switch circuit 715 may be in a turned-on state. When the transistor F_DIAG 815 is turned on, the discharging circuit 714 may be electrically connected to the diagnostic circuit 717.

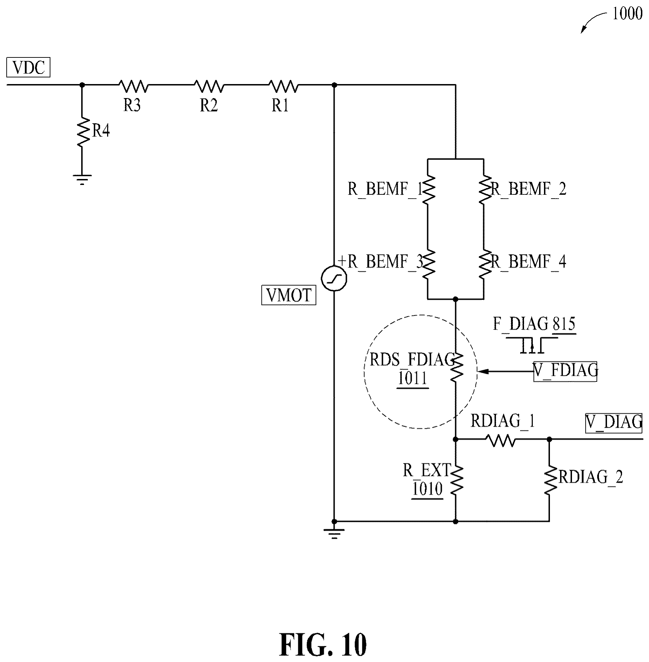

According to an embodiment, the diagnostic circuit 717 may include a plurality of resistors R_EXT, RDIAG_1, and RDIAG_2. When electrically connected to the discharging circuit 714, the diagnostic circuit 717 may output the diagnostic voltage V_DIAG. For example, the voltage VMOT may be voltage-divided by the plurality of resistors R_EXT, RDIAG_1, and RDIAG_2 of the diagnostic circuit 717, the plurality of resistors R_BEMF_1, R_BEMF_2, R_BEMF_3, and R_BEMF_4 of the discharging circuit 714, and a resistor RDS_FDIAG (e.g., the resistor between the source and drain of the transistor F_DIAG 815)(e.g., RDS_FDIAG 1011 of Fig. 10) of the transistor F_DIAG 815. Through this voltage division, the diagnostic circuit 717 may output the diagnostic voltage V_DIAG. In the example illustrated in FIG. 8, the voltage at a node 817 may correspond to the diagnostic voltage V_DIAG. The voltage value of the diagnostic voltage V_DIAG may be expressed, for example, by Equation 2 below.

[Equation 2]

$$V\_DIAG = \cfrac{\cfrac{VMOT \cdot R\_EXT \cdot RDIAG\_2}{(R\_EXT + RDIAG\_1 + RDIAG\_2)}}{\cfrac{(R\_BEMF\_1 + R\_BEMF\_3) \cdot (R\_BEMF\_2 + R\_BEMF\_4)}{R\_BEMF\_1 + R\_BEMF\_2 + R\_BEMF\_3 + R\_BEMF\_4} + RDS\_FDIAG + \cfrac{R\_EXT \cdot (RDIAG\_1 + RDIAG\_2)}{R\_EXT + RDIAG\_1 + RDIAG\_2}}$$

According to an embodiment, the processor 712 may receive the diagnostic voltage V_DIAG from the diagnostic circuit 717. Based on the voltage value of the diagnostic voltage V_DIAG, the processor 712 may determine whether the discharging circuit 714 is in an abnormal state. For example, the processor 712 may determine that the discharging circuit 714 is in a normal state when the voltage value of the diagnostic voltage V_DIAG corresponds to a predetermined voltage value (or normal voltage value) at a given voltage VMOT (or voltage VDC). The processor 712 may determine that the discharging circuit 714 is in a first abnormal state when the voltage value of the diagnostic voltage V_DIAG is 0 at a given voltage VMOT (or voltage VDC). The processor 712 may determine that the discharging circuit 714 is in a second abnormal state when the voltage value of the diagnostic voltage V_DIAG is greater than the predetermined voltage value (or normal voltage value) at a given voltage VMOT (or voltage VDC).

According to an embodiment, the wearable apparatus 700 may include a temperature sensor (not shown) (e.g., a negative temperature coefficient (NTC) thermistor) capable of measuring the temperature of the transistor F_BEMF 816-2. The processor 712 may receive the temperature value of the transistor F_BEMF 816-2 from the temperature sensor. When the received temperature value exceeds a threshold temperature value, the processor 712 may determine that the discharging circuit 714 is in a second abnormal state.

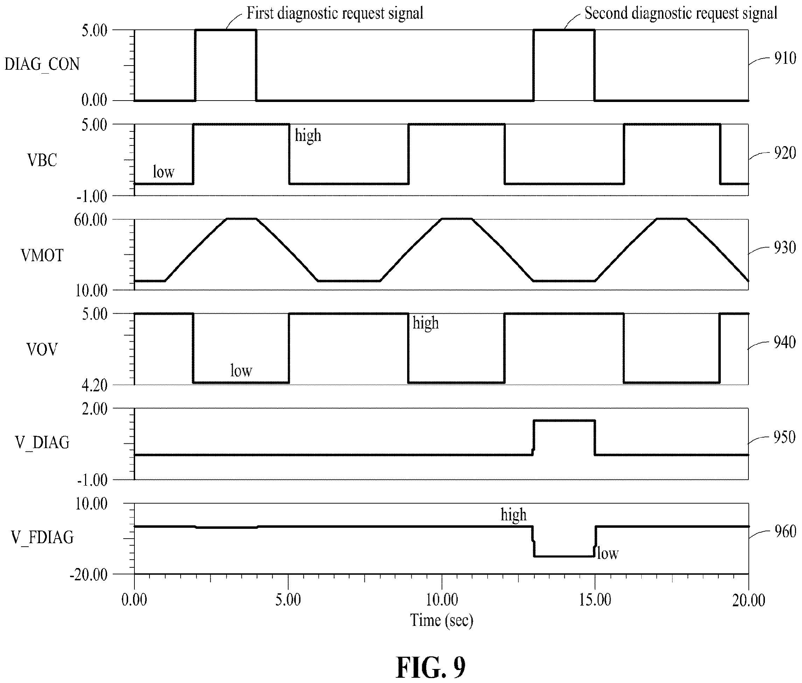

FIG. 9 illustrates an example of a diagnostic operation of a wearable apparatus, according to an embodiment.

FIG. 9 illustrates a waveform 910 of the diagnostic request signal DIAG_CON, a waveform 920 of the voltage VBC, a waveform 930 of the voltage VMOT, a waveform 940 of the voltage VOV, a waveform 950 of the diagnostic voltage V_DIAG, and a waveform 960 of the voltage V_FDIAG.

As shown in the waveform 910 of FIG. 9, the processor 712 may generate a first diagnostic request signal. For example, the processor 712 may generate a first diagnostic request signal when the period of the diagnostic request signal arrives or when the processor 712 receives a diagnostic request.

When the first diagnostic request signal is generated, an overvoltage state may occur. As shown in the waveform 940, in the overvoltage state, the voltage VOV may have a low value (e.g., 4.2 V) (VOV=low). When VOV=low, the transistor TR1 816-1 may be turned on. As a result, as shown in the waveform 920, the voltage VBC may have a high value (e.g., 5 V) (VBC=high). When VBC=high, the transistor F_BEMF 816-2 may be turned on and the discharging circuit 714 may consume the motor electromotive force. According to an embodiment, instead of the transistor TR1 816-1, a transistor that is different from the transistor TR1 816-1 may be used, and/or instead of the transistor F_BEMF 816-2, a transistor that is different from the transistor F_BEMF 816-2 may be used. In this case, when VOV=high and VBC=low (or when VOV=high/VBC=high or VOV=low/VBC=low), the discharging circuit 714 may consume the motor electromotive force.

The first diagnostic request signal generated by the processor 712 may be transmitted to the control circuit 713. When the processor 712 generates the first diagnostic request signal in the overvoltage state, the processor 712 may control the control circuit 713 such that the transistor F_DIAG 815 is not turned on, even when the control circuit 713 receives the first diagnostic request signal. When the first diagnostic request signal is generated in the overvoltage state, as shown in the waveform 960, a voltage (e.g., a voltage V_FDIAG with a low value) that is sufficient to turn on the transistor F_DIAG 815 may not be applied to the gate of the transistor F_DIAG 815. As described below, when the motor 718 is in the overvoltage state, the processor 712 may determine whether the discharging circuit 714 is in an abnormal state through the change in the voltage VDC.

The processor 712 may generate a second diagnostic request signal. For example, the processor 712 may generate the second diagnostic request signal when the period of the diagnostic request signal arrives or when the processor 712 receives a diagnostic request.

When the second diagnostic request signal is generated, the overvoltage state may not occur. As shown in the waveform 940, in the overvoltage state, the voltage VOV may have a high value (e.g., 5 V), and as shown in the waveform 920, the voltage VBC may have a low value (e.g., about 0 V).

The processor 712 may transmit the second diagnostic request signal to the control circuit 713. When the second diagnostic request signal is generated in a non-overvoltage state, the processor 712 may control the control circuit 713 such that the control circuit 713 may turn on the transistor F_DIAG 815. When the second diagnostic request signal is generated in a non-overvoltage state, as shown in the waveform 960, the control circuit 713 may apply, to the gate of the transistor F_DIAG 815, a voltage (e.g., the voltage V_FDIAG with a low value (e.g., about 0 V)) that is sufficient to turn on the transistor F_DIAG 815. According to an embodiment, a different type of transistor may be used instead of the transistor F_DIAG 815. In this case, the different type of transistor may be turned on when V_FDIAG=high.

When the transistor F_DIAG 815 is turned on, the diagnostic circuit 717 may be electrically connected, directly or indirectly, to the discharging circuit 714, and the diagnostic circuit 717 may output the diagnostic voltage V_DIAG to the processor 712.

The processor 712 may determine whether the discharging circuit 714 is in an abnormal state based on the voltage value of the diagnostic voltage V_DIAG.

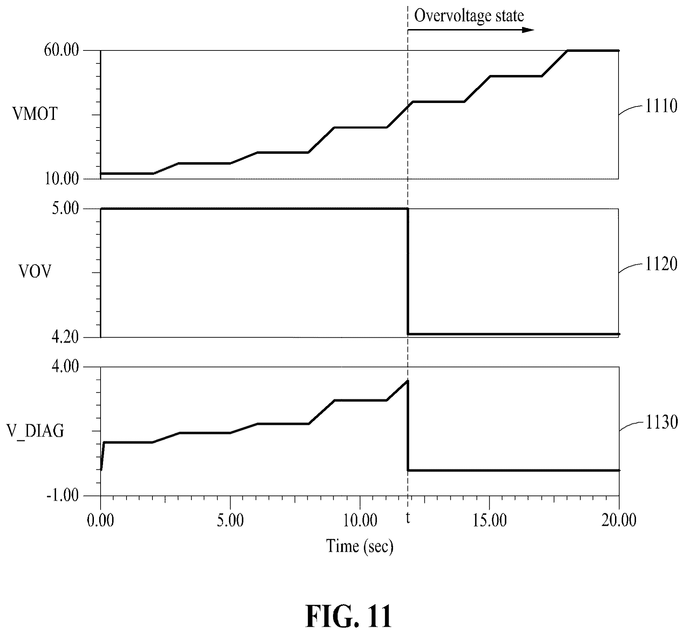

FIGS. 10 and 11 are diagrams illustrating examples of a diagnostic operation of a wearable apparatus when a motor electromotive force of the wearable apparatus is not consumed, according to an embodiment.

In FIG. 10, an example of an equivalent circuit 1000 of the circuit 800 of FIG. 8 is illustrated when the motor electromotive force of the wearable apparatus 700 is not consumed by the discharging circuit 714 (or when the motor 718 is not in an overvoltage state).

In the example illustrated in FIG. 10, the voltage VMOT may be the voltage of the battery 720 applied to the motor 718, for example. For example, in a pre-exercise state (or post-exercise state), the wearable apparatus 700 may provide the power of the battery 720 to the motor 718 to determine whether the discharging circuit 714 is in a normal state. The voltage of the battery 720 may be applied to the motor 718.

According to an embodiment, the resistance value of a resistor R_EXT 1010 may be determined to satisfy a first condition, which lowers the power consumption of the battery 720, and a second condition, which distinguish between the diagnostic voltage value when the discharging circuit 714 is in an abnormal state and the diagnostic voltage value when the discharging circuit 714 is in a normal state. For example, when the resistance value of the resistor R_EXT 1010 is low, relatively a lot of currents may flow through the resistors R_BEMF_1, R_BEMF_2, R_BEMF_3, and R_BEMF_4, and power consumption of the battery 720 may be high due to these resistors R_BEMF_1, R_BEMF_2, R_BEMF_3, and R_BEMF_4. When the resistance value of the resistor R_EXT 1010 is high, the ratio between the equivalent resistance of the resistors R_BEMF_1, R_BEMF_2, R_BEMF_3, and R_BEMF_4 and the resistor R_EXT 1010 may increase. As a result, the difference between the diagnostic voltage value when the discharging circuit 714 is in an abnormal state and the diagnostic voltage value when the discharging circuit 714 is in a normal state may be negligible. It may be difficult to distinguish between the diagnostic voltage value when the discharging circuit 714 is in an abnormal state and the diagnostic voltage value when the discharging circuit 714 is in a normal state. Therefore, the resistance value of the resistor R_EXT 1010 may be determined to an appropriate level such that the power consumption of the battery 720 is lowered and the diagnostic voltage value when the discharging circuit 714 is in the abnormal is distinguished from the diagnostic voltage value when the discharging circuit 714 is in the normal state. In an embodiment to be described below, the resistance value of the resistor R_EXT 1010 may be, for example, 4 kiloohms (kΩ) but is not limited thereto.

In the example illustrated in FIG. 10, the resistor R_EXT 1010 may be a single resistor but is not limited thereto. Resistors connected in series and/or parallel may be used in place of the resistor R_EXT 1010. For example, the resistance value of the resistor R_EXT 1010 may be 4 kΩ. In another example, instead of the resistor R_EXT 1010 of 4 kΩ, two 2k Ω resistors connected, directly or indirectly, in series or two 8 kΩ resistors connected, directly or indirectly, in parallel may be used.

A resistor RDS_FDIAG 1011 may be the resistor between the source and drain of the transistor F_DIAG 815. The resistance value of the resistor RDS_FDIAG 1011 may be, for example, in a range of several milliohms (mΩ). When a voltage (e.g., the voltage V_FDIAG with a low value or V_FDIAG=low) that may turn on the transistor F_DIAG 815 is applied to the gate of the transistor F_DIAG 815, the resistance RDS_FDIAG 1011 may be generated.

According to an embodiment, the processor 712 may generate the diagnostic request signal DIAG_CON when the motor 718 is not in an overvoltage state. The processor 712 may check whether the second switch circuit 716 is in the turned-on state (or operates) by using the voltage value of the voltage VBC when the motor 718 is not in an overvoltage state. For example, the processor 712 may check that the second switch circuit 716 is in the turned-off state when the voltage value of the voltage VBC is low when the motor 718 is not in an overvoltage state. The processor 712 may check that the second switch circuit 716 is in the turned-on state when the voltage value of the voltage VBC is high when the motor 718 is not in an overvoltage state.

According to an embodiment, when the diagnostic request signal DIAG_CON is generated and the motor 718 is not in an overvoltage state, the comparator 811 may not output a first signal (e.g., a voltage signal). The voltage VOV may have a high value. In other words, VOV=high. The processor 712 may periodically generate the diagnostic request signal DIAG_CON to periodically monitor whether the discharging circuit 714 is in an abnormal state. When the diagnostic request signal DIAG_CON (e.g., the first diagnostic request signal in FIG. 9) is generated, the motor 718 may be in an overvoltage state. When the motor 718 is in an overvoltage state, the processor 712 may generate the diagnostic request signal DIAG_CON (e.g., the first diagnostic request signal of FIG. 9). When the motor 718 is in an overvoltage state, as described below with reference to FIGS. 12 to 14, the processor 712 may determine whether the discharging circuit 714 is in an abnormal state through a change in the voltage VDC.

According to an embodiment, the control circuit 713 may receive the diagnostic request signal DIAG_CON from the processor 712 and output a voltage (e.g., the voltage V_FDIAG with a low value or V_FDIAG = low) that may turn on the transistor F_DIAG 815 to the first switch circuit 715. As described in the previous example, when the voltage V_FDIAG with a low value is applied to the gate of the transistor F_DIAG 815 of the first switch circuit 715, the discharging circuit 714 may be electrically connected, directly or indirectly, to the diagnostic circuit 717.

According to an embodiment, when the discharging circuit 714 is electrically connected, directly or indirectly, to the diagnostic circuit 717, the voltage VMOT may be distributed to the resistors R_BEMF_1, R_BEMF_2, R_BEMF_3, and R_BEMF_4, the resistor RDS_FDIAG 1011, and the resistor R_EXT 1010 of the discharging circuit 714. The diagnostic voltage V_DIAG may be determined by the voltage and the resistors RDIAG_1 and RDIAG_2 applied to the resistor R_EXT 1010. For example, the voltage value of the diagnostic voltage V_DIAG may be expressed by Equation 2 above.

According to an embodiment, the processor 712 may receive the diagnostic voltage V_DIAG from the diagnostic circuit 717. The processor 712 may convert the diagnostic voltage V_DIAG into a digital signal through an analog to digital converter (ADC). Based on the voltage value of the diagnostic voltage V_DIAG, the processor 712 may determine whether the discharging circuit 714 is in an abnormal state. An embodiment in which the processor 712 determines whether the discharging circuit 714 is in an abnormal state based on the voltage value of the diagnostic voltage V_DIAG is described with reference to FIG. 11.

According to an embodiment, the resistance value of the resistor R_EXT 1010 may be greater than the resistance value of each of the resistors R_BEMF_1, R_BEMF_2, R_BEMF_3, and R_BEMF_4.

FIG. 11 illustrates a waveform 1110 of the voltage VMOT, a waveform 1120 of the voltage VOV, and a waveform 1130 of the diagnostic voltage V_DIAG.

In the example illustrated in FIG. 11, the resistance value of each of the resistors R_BEMF_1, R_BEMF_2, R_BEMF_3, and R_BEMF_4 of FIG. 10 may be, for example, 50 Ω, the resistance value of the resistor RDS_FDIAG 1011 of FIG. 10 may be, for example, 10 mΩ, the resistance value of the resistor R_EXT 1010 may be, for example, 4 kΩ, the resistance value of the resistor RDIAG_1 may be, for example, 100 kΩ, and the resistance value of the resistor RDIAG_2 may be, for example, 10 kΩ.

In the example illustrated in FIG. 11, a voltage at which the wearable apparatus 700 may operate by receiving the power of the battery 720 or a motor electromotive force may be, for example, 16 V, the minimum voltage may be 12 V, and the reference voltage VREF may be 40 V.

In the example illustrated in FIG. 11, at a time point t, the motor 718 may be in an overvoltage state.

When the motor 718 is not in an overvoltage state, the voltage VOV may have a high value. For example, the comparison circuit 711 may not output the first voltage signal when the motor 718 is not in an overvoltage state. As a result, the voltage VOV may have a high value. The comparison circuit 711 may control the second switch circuit 716 so that the voltage VOV has a high value when the motor 718 is not in an overvoltage state. When the motor 718 is in an overvoltage state, the comparison circuit 711 may output the first signal, causing the voltage VOV to have a low value. The comparison circuit 711 may control the second switch circuit 716 so that the voltage VOV has a low value when the motor 718 is in an overvoltage state.

The processor 712 may receive the diagnostic voltage V_DIAG from the diagnostic circuit 717 and may determine whether the discharging circuit 714 is in an abnormal state based on the voltage value of the diagnostic voltage V_DIAG.

Table 1 below shows examples of the normal voltage values of the diagnostic voltage V_DIAG at the voltage VMOT (or voltage VDC), the voltage values of the diagnostic voltage V_DIAG when the discharging circuit 714 is in a first abnormal state, and the voltage values of the diagnostic voltage V_DIAG when the discharging circuit 714 is in a second abnormal state.

[Table 1]

| Voltage VMOT (or voltage VDC) | Diagnostic voltage V_DIAG | | |
|---|---|---|---|
| | Normal state | First abnormal state | Second abnormal state |
| 12 V | 1.077 V | 0 V | 1.091 V |
| 16 V | 1.436 V | 0 V | 1.455 V |
| 20 V | 1.795 V | 0 V | 1.818 V |
| 30 V | 2.692 V | 0 V | 2.727 V |
| 40 V | - | - | - |
| 50 V | - | - | - |

When the voltage VMOT is 12 V, the normal voltage value may be, for example, 1.077 V, when the voltage VMOT is 16 V, the normal voltage value may be, for example, 1.436 V, when the voltage VMOT is 20 V, the normal voltage value may be, for example, 1.795 V, and when the voltage VMOT is 30 V, the normal voltage value may be, for example, 2.692 V.

At the voltage VMOT, the processor 712 may determine that the discharging circuit 714 is in a normal state when the voltage value of the diagnostic voltage V_DIAG corresponds to the normal voltage value. At the voltage VMOT, the processor 712 may determine that the discharging circuit 714 is in the first abnormal state when the voltage value of the diagnostic voltage V_DIAG corresponds to 0. At the voltage VMOT, the processor 712 may determine that the discharging circuit 714 is in the second abnormal state when the voltage value of the diagnostic voltage V_DIAG is greater than the normal voltage value. For example, at VMOT=16 V, the processor 712 may determine that the discharging circuit 714 is in a normal state when the voltage value of the diagnostic voltage V_DIAG corresponds to the normal voltage value (e.g., 1.436 V). At VMOT=16 V, the processor 712 may determine that the discharging circuit 714 is in the first abnormal state when the voltage value of the diagnostic voltage V_DIAG corresponds to 0 V. At VMOT=16 V, the processor 712 may determine that the discharging circuit 714 is in the second abnormal state when the voltage value of the diagnostic voltage V_DIAG corresponds to a value (e.g., 1.455 V) greater than the normal voltage value.

According to an embodiment, when VMOT is 40 V or higher, an overvoltage state may occur, and the diagnostic circuit 717 may not output the diagnostic voltage V_DIAG. When in an overvoltage state, the processor 712 may determine whether the discharging circuit 714 is in an abnormal state based on at least one of a change in the voltage VDC or the current value of the current in the discharging circuit 714. This will be described with reference to FIGS. 12, 13, and 14.

FIGS. 12, 13, and 14 are diagrams illustrating examples of an diagnostic operation of a wearable apparatus in a state in which the electromotive force of a motor of the wearable apparatus is consumed, according to an embodiment.

FIG. 12 illustrates an example of an equivalent circuit 1200 of the circuit 800 of FIG. 8 when the motor electromotive force of the wearable apparatus 700 is consumed by the discharging circuit 714 (or when the motor 718 is in an overvoltage state).

In the example illustrated in FIG. 12, the voltage VMOT may represent the voltage of the motor electromotive force. For example, in a resistance mode, the wearable apparatus 700 may provide the motor electromotive force to the motor 718 such that a resistance force may be provided to a user. The voltage of the motor electromotive force may be applied to the motor 718.

A resistor RDS_FBEMF 1210 may be the resistor between the source and drain of the transistor F_BEMF 816-2. When the voltage VBC (e.g., the voltage VBC with a high value or VBC=high) that may turn on the transistor F_BEMF 816-2 is applied to the gate of the transistor F_BEMF 816-2, the resistor RDS_FBEMF 1210 may be generated.

When the voltage VOV has a low value, the voltage VBC may have a high value, and the voltage VBC with a high value may be applied to the gate of the transistor F_BEMF 816-2. In this case, the transistor F_BEMF 816-2 may be turned on. When the transistor F_BEMF 816-2 is turned on, the discharging circuit 714 may consume the motor electromotive force through the plurality of resistors R_BEMF_1, R_BEMF_2, R_BEMF_3, and R_BEMF_4.

The processor 712 may receive the voltage VOV from the second switch circuit 716 and may detect (or determine) whether an overvoltage state occurs based on the voltage value of the voltage VOV. For example, the processor 712 may detect (or determine) that an overvoltage state occurs when the voltage VOV has a low value. The processor 712 may detect (or determine) that an overvoltage state does not occur when the voltage VOV has a high value.

The processor 712 may receive the voltage VBC from the second switch circuit 716 and may check (or determine) whether the discharging circuit 714 consumes the motor electromotive force based on the voltage value of the voltage VBC. For example, the processor 712 may check (or determine) that the discharging circuit 714 does not consume the motor electromotive force when the voltage VBC has a low value. The processor 712 may check (or determine) that the discharging circuit 714 consumes the motor electromotive force when the voltage VBC has a high value.

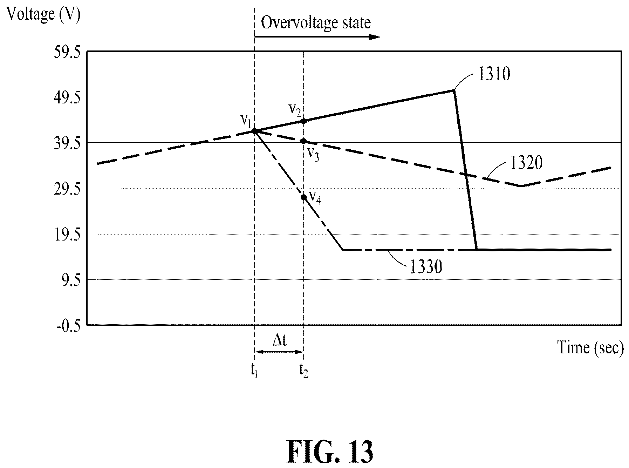

The processor 712 may receive the voltage VDC from the comparison circuit 716 and may determine whether the discharging circuit 714 is in an abnormal state based on a change (or a change pattern of the voltage VDC) in the voltage VDC after an overvoltage state occurs. This will be described with reference to FIG. 13.

FIG. 13 illustrates a graph 1310 of VDC changes when the discharging circuit 714 is in a first abnormal state, a graph 1320 of VDC changes when the discharging circuit 714 is in a normal state, and a graph 1330 of VDC changes when the discharging circuit 714 is in a second abnormal state.

In the example illustrated in FIG. 13, a resistance value of each of the resistors R_BEMF_1, R_BEMF_2, R_BEMF_3, and R_BEMF_4 of FIG. 12 may be, for example, 50 $\Omega$, and the resistance value of the resistor RDS_FBEMF 1210 of FIG. 12 may be, for example, 10 m$\Omega$.

At a time point $t_1$ in FIG. 13, the motor 718 may be in an overvoltage state.

The processor 712 may store, in a memory (or a buffer) (not shown), the voltage value (e.g., a voltage value $V_1$ of FIG. 13) of the voltage VDC when the motor 718 is in an overvoltage state (or when VOV=low or VBC=high).

The processor 712 may determine whether the discharging circuit 714 is in an abnormal state based on a change in VDC (or a change pattern of VDC) during a predetermined time interval (e.g., the time interval $\triangle t$ from the time point $t_1$ to a time point $t_2$).

For example, the processor 712 may obtain the voltage value (e.g., a voltage value $V_3$) of the voltage VDC at the time point $t_2$. The processor 712 may determine that the voltage VDC decreases during the time interval $\triangle t$ based on the voltage values $V_1$ and $V_3$. In an overvoltage state, the discharging circuit 714 may consume the motor electromotive force, and as the motor electromotive force is consumed, the voltage VMOT and the voltage VDC may decrease. The processor 712 may determine whether the discharging circuit 714 is in an abnormal state based on the amount of change (or decrease) in the voltage VDC during a predetermined time interval in a state in which the motor electromotive force is consumed by the discharging circuit 714. The processor 712 may determine whether the amount of change (or decrease) in VDC over $\triangle t$ (e.g., $V_3 - V_1$) corresponds to a predetermined level (e.g., a normal level of voltage change (or decrease)). The predetermined level (e.g., a normal level of voltage change (or decrease)) may be determined, for example, by at least one or two or more or all of the resistance values of the resistors (e.g., the resistors R_BEMF_1, R_BEMF_2, R_BEMF_3, R_BEMF_4, and RDS_FBEMF). When the amount of change in VDC over $\triangle t$ corresponds to the predetermined level, the processor 712 may determine that the discharging circuit 714 is in a normal state.

In another example, the processor 712 may obtain the voltage value (e.g., a voltage value $V_4$) of the voltage VDC at the time point $t_2$. The processor 712 may determine that the voltage VDC decreases during the time interval $\triangle t$ based on the voltage values $V_1$ and $V_4$. The processor 712 may determine that the discharging circuit 714 is in a second abnormal state because the amount of change (or decrease) in VDC over $\triangle t$ (e.g., $V_4 - V_1$) may exceed the predetermined level (e.g., a normal level of voltage change (or decrease)). When at least one of the resistors R_BEMF_1, R_BEMF_2, R_BEMF_3, and R_BEMF_4 of the discharging circuit 714 is in a short state and/or at least one of the wire harnesses 814-1 and 814-2 is in a short state, a current with a relatively high intensity may flow through the discharging circuit 714. As a result, the amount of change (or decrease) in VDC over $\triangle t$ (e.g., $V_4$ - $V_1$) may exceed a predetermined level, and the processor 712 may determine that the discharging circuit 714 is in a second abnormal state when the amount of change (or decrease) in VDC over $\triangle t$ (e.g., $V_4$ - $V_1$) exceeds the predetermined level.

In another example, the processor 712 may obtain the voltage value (e.g., the voltage value $V_2$) of the voltage VDC at the time point $t_2$. The processor 712 may determine that the voltage VDC increases over $\triangle t$ based on the voltage values $V_1$ and $V_2$. The processor 712 may determine that the amount of change (or increase) in VDC over $\triangle t$ (e.g., $V_2$ - $V_1$) is greater than 0. When the motor electromotive force is not consumed by the discharging circuit 714 in an overvoltage state, the voltage VDC may increase. The processor 712 may determine that the discharging circuit 714 is in the first abnormal state when the voltage VDC increases during a predetermined time interval (e.g., $\triangle t$).

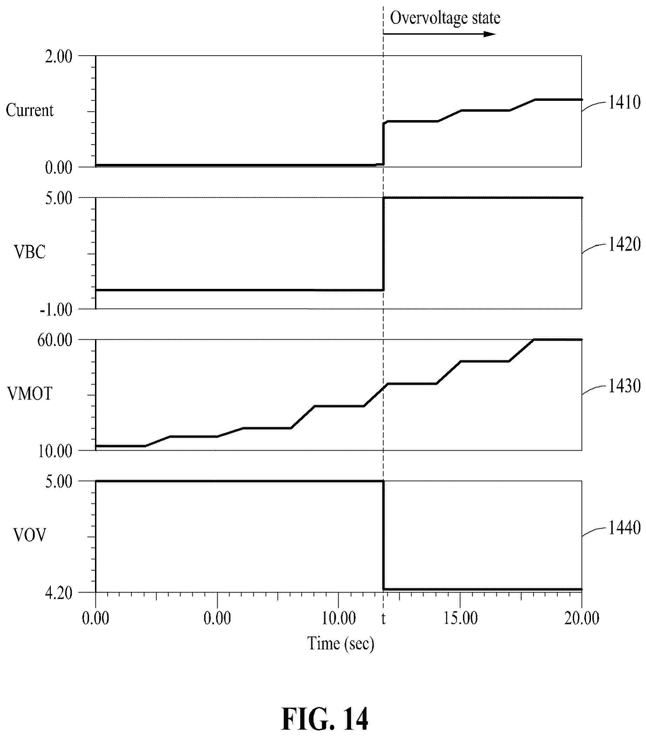

The processor 712 may receive the current value of the current in the discharging circuit 714 from a current sensor (not shown) and may determine whether the discharging circuit 714 is in an abnormal state based on the received current value. This will be described with reference to FIG. 14.

FIG. 14 illustrates a waveform 1410 of the current in the discharging circuit 714, a waveform 1420 of the voltage VBC, a waveform 1430 of the voltage VMOT, and a waveform 1440 of the voltage VOV).

In the example illustrated in FIG. 14, at the time point t, the motor 718 may be in an overvoltage state.

When the motor 718 is in an overvoltage state, the processor 712 may determine whether the discharging circuit 714 is in an abnormal state based on the current value (e.g., the current value of the current in the discharging circuit 714) received from the current sensor. Table 2 below shows examples of the current values of the current in the discharging circuit 714 in a normal state, the current values of the current in the discharging circuit 714 in a first abnormal state, and the current values of the current in the discharging circuit 714 in a second abnormal state, respectively, at the voltage VMOT (or voltage VDC).

[Table 2]

| Voltage VMOT (or voltage VDC) | Current of the discharging circuit 714 | | |
|---|---|---|---|
| | Normal state | First abnormal state | Second abnormal state |
| 12 V | 0 amperes (A) | 0 A | 0 A |
| 16 V | 0 A | 0 A | 0 A |
| 20 V | 0 A | 0 A | 0 A |
| 30 V | 0 A | 0 A | 0 A |
| 40 V | 0.799 A | 0 A | $\infty$ |
| 50 V | 0.999 A | 0 A | $\infty$ |

When the voltage VMOT is 40 V, the normal current value of the discharging circuit 714 may be, for example, 0.799 A, and when the voltage VMOT is 50 V, the normal current value of the discharging circuit 714 may be, for example, 0.999 A.

At the voltage VMOT in an overvoltage state, the processor 712 may determine that the discharging circuit 714 is in a normal state when the current value received from the current sensor corresponds to the normal current value. At the voltage VMOT in an overvoltage state, the processor 712 may determine that the discharging circuit 714 is in a first abnormal state when the received current value corresponds to 0. At the voltage VMOT in an overvoltage state, the processor 712 may determine that the discharging circuit 714 is in a second abnormal state when the received current value exceeds the normal current value by a predetermined level. For example, at VMOT=40 V, the processor 712 may determine that the discharging circuit 714 is in a normal state when the received current value (e.g., the current value of the current in the discharging circuit 714) corresponds to the normal current value (e.g., 0.799 A). At VMOT=40V, the processor 712 may determine that the discharging circuit 714 is in the first abnormal state when the received current value (e.g., the current value of the current in the discharging circuit 714) corresponds to 0 A. At VMOT=40V, the processor 712 may determine that the discharging circuit 714 is in the second abnormal state when the received current value (e.g., the current value of the current in the discharging circuit 714) exceeds the normal current value by a predetermined level.

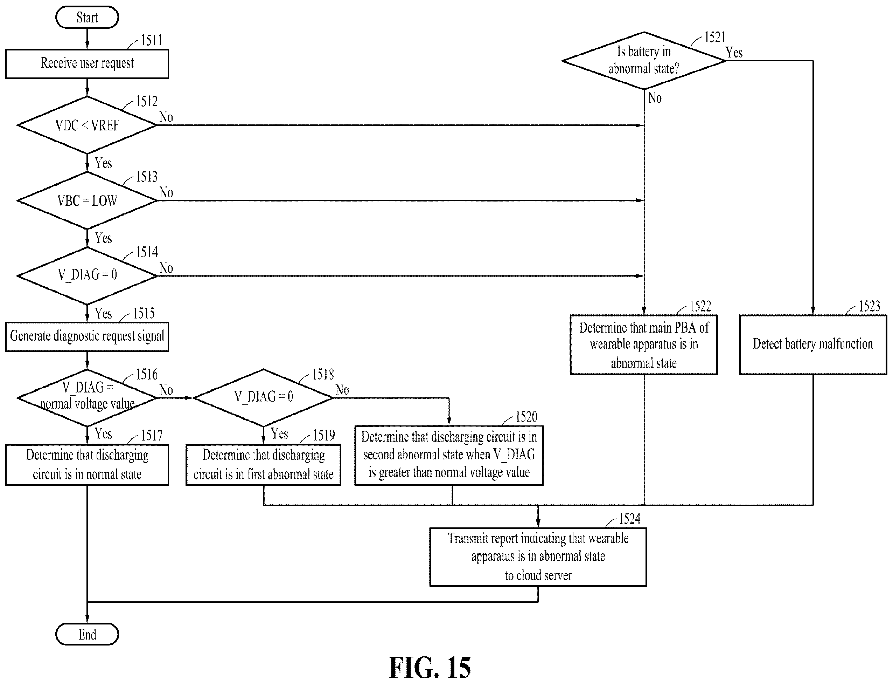

FIG. 15 is a flowchart illustrating an example of a diagnostic operation when a wearable apparatus is in a pre-exercise state or a post-exercise state of a user, according to an embodiment.

Referring to FIG. 15, in operation 1511, the wearable apparatus 700 may receive a user request (e.g., a request to start or stop driving the wearable apparatus 700) from at least one of the user, the electronic device 210, or the other wearable apparatus 220.

The wearable apparatus 700 may determine that an operation state (or a state of the user) is a pre-exercise state when there is a request to start driving the wearable apparatus 700. According to an embodiment, the request to start driving the wearable apparatus 700 may include, for example, information indicating an operation mode (e.g., a resistance mode) of the wearable apparatus 700 and/or information about the magnitude of external force that the wearable apparatus 700 provides to the user.

The wearable apparatus 700 may determine that the operation state (or the state of the user) is a post-exercise state when there is a request to stop driving the wearable apparatus 700. The request to stop driving may include, for example, a user input to turn off the power of the wearable apparatus 700.

In both the pre-exercise state and the post-exercise state, the wearable apparatus 700 may not provide external force to the user.

The wearable apparatus 700 may initiate (or perform) a diagnostic operation by providing the power from the battery 720 to the motor 718 when there is the user request (e.g., a request to start driving the wearable apparatus 700 or a request to stop driving the wearable apparatus 700). In both the pre-exercise state and the post-exercise state, the voltage of the battery 720 may correspond to the voltage VMOT used by the motor 718 (or the voltage applied to the motor 718).

The wearable apparatus 700 may generate the voltage VDC based on the voltage VMOT. For example, the wearable apparatus 700 may input the voltage VMOT into a voltage dividing circuit and generate the voltage VDC through the voltage dividing circuit. The wearable apparatus 700 may convert the voltage VMOT to the voltage VDC using a voltage dividing technique. In another example, the wearable apparatus 700 may input the voltage VMOT into an OP AMP and generate VDC through the OP AMP. The wearable apparatus 700 may convert the voltage VMOT into the voltage VDC using the OP AMP.

In operation 1512, the wearable apparatus 700 may determine whether the voltage VDC is less than the reference voltage VREF.

When the voltage VDC is less than the reference voltage VREF (operation 1512-Yes), the wearable apparatus 700 may determine in operation 1513 whether the voltage VBC is low. The wearable apparatus 700 may determine whether the voltage VBC has a low value.

When the voltage VBC is low (operation 1513-Yes), the wearable apparatus 700 may determine in operation 1514 whether the diagnostic voltage V_DIAG is 0.

When the diagnostic voltage V_DIAG is 0 (operation 1514-Yes), the wearable apparatus 700 may generate a diagnostic request signal in operation 1515.

When generating a diagnostic request signal, the wearable apparatus 700 may determine in operation 1516 whether the diagnostic voltage V_DIAG has a normal voltage value (for example, in Table 1, the normal voltage value is 1.077 V when VMOT=12 V, 1.436 V when VMOT=16 V, 1.795V when VMOT=20 V, 2.692 V when VMOT=30V, etc.).

When the diagnostic voltage V_DIAG has a normal voltage value (operation 1516-Yes), the wearable apparatus 700 may determine in operation 1517 that the discharging circuit 714 is in a normal state. When determining that the discharging circuit 714 is in a normal state, the wearable apparatus 700 may perform an operation according to the user request. For example, when there is a request to start driving the wearable apparatus 700 in the pre-exercise state, the wearable apparatus 700 may start to provide external force to the user according to the request. By starting to provide external force to the user, the wearable apparatus 700 may be in an exercising state (or an exercise state). When there is a request to stop driving the wearable apparatus 700 in the post-exercise state, the wearable apparatus 700 may turn off the power according to the request.

When the diagnostic voltage V_DIAG does not have a normal voltage value (operation 1516-No), the wearable apparatus 700 may determine in operation 1518 whether the diagnostic voltage V_DIAG is 0.

When the diagnostic voltage V_DIAG is 0 (operation 1518-Yes), the wearable apparatus 700 may determine in operation 1519 that the discharging circuit 714 is in a first abnormal state (e.g., an electrical open state of a resistor of the discharging circuit 714 and/or a state in which a wire harness of the discharging circuit 714 is disconnected). When the discharging circuit 714 is in the first abnormal state, the wearable apparatus 700 may transmit, to a cloud server, a report indicating that the wearable apparatus 700 is in an abnormal state (e.g., the first abnormal state) in operation 1524.

When the diagnostic voltage V_DIAG is not 0 (operation 1518-No) and the diagnostic voltage V_DIAG is greater than a normal voltage value, the wearable apparatus 700 may determine in operation 1520 that the discharging circuit 714 is in the second abnormal state (e.g., the electrical short state of the resistor of the discharging circuit 714 and/or a state in which the wire harness of the discharging circuit 714 is shorted). For example, when VMOT=12 V, the normal voltage value may be 1.077 V and the voltage value of the diagnostic voltage V_DIAG may be 1.091 V. In this case, the wearable apparatus 700 may check that the voltage value of the diagnostic voltage V_DIAG is greater than the normal voltage value and determine that the discharging circuit 714 is in the second abnormal state. When the discharging circuit 714 is in the second abnormal state, the wearable apparatus 700 may transmit a report indicating that the wearable apparatus 700 is in an abnormal state (e.g., the second abnormal state) to the cloud server in operation 1524.

Returning to operation 1512, when determining that VDC is greater than or equal to VREF (operation 1512-No), the wearable apparatus 700 may determine in operation 1521 whether the battery 720 is in an abnormal state. For example, when the battery 720 is in an abnormal state such as when there is an issue with a protection circuit of the battery 720, an excessive voltage (or an excessive current) may be generated by the battery 720. In this case, VDC may be greater than or equal to VREF. When VDC is greater than or equal to VREF, the wearable apparatus 700 may determine whether the battery 720 is in an abnormal state.

When the battery 720 is in a normal state (operation 1521-No), the wearable apparatus 700 may determine in operation 1522 that a main PBA of the wearable apparatus 700 is in an abnormal state. The main PBA may be a PCB in which, for example, the processor 712 comprising processing circuitry, the communication module 721 comprising communication circuitry, and the like may be located. In operation 1524, the wearable apparatus 700 may transmit, to the cloud server, a report (e.g., a report indicating that the main PBA of the wearable apparatus 700 is in an abnormal state) indicating that the wearable apparatus 700 is in an abnormal state.

When the battery is in an abnormal state (operation 1521-Yes), the wearable apparatus 700 may detect a battery failure in operation 1523. In operation 1524, the wearable apparatus 700 may transmit, to the cloud server, a report (e.g., a report indicating that a battery malfunction) indicating that the wearable apparatus 700 is in an abnormal state.

The embodiments described with reference to FIGS. 1 to 14 may apply to the embodiments of FIG. 15.

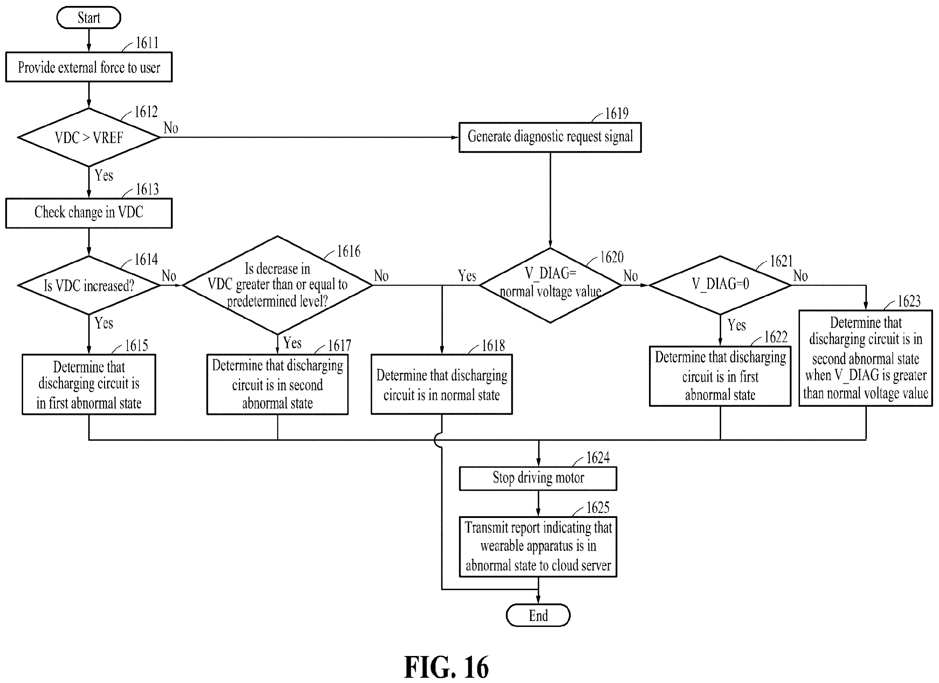

FIG. 16 is a flowchart illustrating an example of a diagnostic operation when a wearable apparatus is in an exercising state of a user, according to an embodiment.

Referring to FIG. 16, in operation 1611, the wearable apparatus 700 may provide external force to the user. The exercising state (or an exercise state) may include, for example, a state in which the wearable apparatus 700 provides external force to the user.

In the exercising state, motor electromotive force may be generated by the movement of the user, and the voltage of the generated motor electromotive force may be applied to the motor 718. In the exercising state, the voltage of the motor electromotive force may correspond to the voltage VMOT (or the voltage applied to the motor 718) used by the motor 718.

The wearable apparatus 700 may generate the voltage VDC based on the voltage VMOT. For example, the wearable apparatus 700 may input the voltage VMOT into a voltage dividing circuit and generate the voltage VDC through the voltage dividing circuit. The wearable apparatus 700 may convert the voltage VMOT to the voltage VDC using a voltage division technique. In another example, the wearable apparatus 700 may input the voltage VMOT into an OP AMP and generate the voltage VDC through the OP AMP. The wearable apparatus 700 may convert the voltage VMOT to the voltage VDC through the OP AMP.

In operation 1612, the wearable apparatus 700 may determine whether the voltage VDC exceeds the reference voltage VREF.

When the voltage VDC exceeds the reference voltage VREF (operation 1612-Yes), the wearable apparatus 700 may check a change in the voltage VDC in operation 1613. For example, the wearable apparatus 700 may check the change in the voltage VDC based on the voltage value of the voltage VDC at a second time point (e.g., the time point $t_2$ of FIG. 13) and the voltage value (e.g., $V_1$ of FIG. 13) of the voltage VDC at a first time point (e.g., the time point $t_1$ of FIG. 13).

In operation 1614, the wearable apparatus 700 may determine whether the voltage VDC increases. For example, the wearable apparatus 700 may determine that the voltage VDC increases when the voltage value (e.g., $V_2$ of FIG. 13) at the second time point is greater than the voltage value (e.g., $V_1$ in FIG. 13) at the first time point.

When determining that the voltage VDC increases (operation 1014-Yes), the wearable apparatus 700 may determine in operation 1615 that the discharging circuit 714 is in a first abnormal state.

When determining that the voltage VDC does not increase (operation 1614-No), the wearable apparatus 700 may determine in operation 1616 whether the decrease in the voltage VDC is greater than or equal to a predetermined level. For example, the wearable apparatus 700 may determine whether the amount of change in the VDC over $\triangle t$ (e.g., the difference between the second time point and the first time point) corresponds to the predetermined level.

When determining that the decrease in the voltage VDC is greater than or equal to the predetermined level (operation 1616-Yes), the wearable apparatus 700 may determine in operation 1617 that the discharging circuit 714 is in a second abnormal state. For example, as described with reference to FIG. 13, the wearable apparatus 700 may determine that the discharging circuit 714 is in the second abnormal state when the amount of change (e.g., $V_4 - V_1$) in the VDC (or the amount of decrease in the VDC) over $\triangle t$ exceeds the predetermined level.

When determining that the decrease in the voltage VDC is at the determined level (operation 1616-No), the wearable apparatus 700 may determine in operation 1618 that the discharging circuit 714 is in a normal state. For example, as described with reference to FIG. 13, the wearable apparatus 700 may determine that the discharging circuit 714 is in a normal state when the amount of change (e.g., $V_3 - V_1$) in VDC over $\triangle t$ corresponds to the predetermined level.

In operation 1612, the wearable apparatus 700 may determine that the voltage VDC does not exceed the reference voltage VREF. In this case, the wearable apparatus 700 may generate a diagnostic request signal in operation 1619. For example, the wearable apparatus 700 may generate the diagnostic request signal based on a diagnostic request received from the electronic device 210, which is in wireless communication connection with the wearable apparatus 700, or may generate the diagnostic request signal when the period for the diagnostic request signal arrives.

After generating the diagnostic request signal, the wearable apparatus 700 may determine in operation 1620 whether the diagnostic voltage V_DIAG has a normal voltage value.

When the diagnostic voltage V_DIAG has the normal voltage value (operation 1620-Yes), the wearable apparatus 700 may determine in operation 1618 that the discharging circuit 714 is in a normal state.

When the diagnostic voltage V_DIAG does not have the normal voltage value (operation 1620-No), the wearable apparatus 700 may determine in operation 1621 whether the diagnostic voltage V_DIAG is 0.

When the diagnostic voltage V_DIAG is 0 (operation 1621-Yes), the wearable apparatus 700 may determine in operation 1622 that the discharging circuit 714 is in the first abnormal state.

When the diagnostic voltage V_DIAG is not 0 (operation 1621-No) and the diagnostic voltage V_DIAG is greater than the normal voltage value, the wearable apparatus 700 may determine in operation 1623 that the discharging circuit 714 is in the second abnormal state.

When determining that the discharging circuit 714 is in an abnormal state (e.g., the first abnormal state or the second abnormal state), the wearable apparatus 700 may stop driving of the motor in operation 1624 and transmit, to a cloud server, a report indicating that the wearable apparatus 700 is in an abnormal state (e.g., the first abnormal state or the second abnormal state) in operation 1625.

The embodiments described with reference to FIGS. 1 to 14 may apply to the embodiments of FIG. 16.

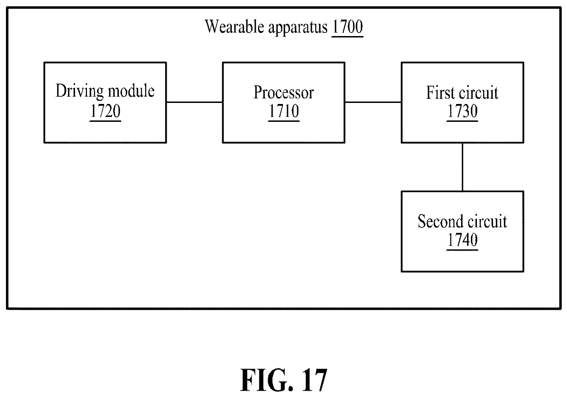

FIG. 17 is a block diagram illustrating an example of a configuration of a wearable apparatus, according to an embodiment.

Referring to FIG. 17, a wearable apparatus 1700 (e.g., the wearable apparatus 100, the wearable apparatus 500, the wearable apparatus 500-1, and the wearable apparatus 700) according to an embodiment may include a processor 1710 (e.g., the processor 512 and/or the processor 712), a driving module 1720 (e.g., the driving modules 35 and 45 and the driving module 530), a first circuit 1730, and a second circuit 1740.

The driving module 1720 may include one or more motors 718.

According to an embodiment, the processor 1710 and the first circuit 1730 may be included in a first PBA, and the second circuit 1740 may be included in a second PBA. The second circuit 1740 may be connected to the first PBA through, for example, one or more wire harnesses.

According to an embodiment, the first circuit 1730 may include one or more electrical components and/or one or more transistors. The first circuit 1730 may include at least some of the conversion circuit 710, the comparison circuit 711, the control circuit 713, the diagnostic circuit 717, the first switch circuit 715, or the second switch circuit 716 or all of them.

According to an embodiment, the second circuit 1740 may include one or more resistors. The second circuit 1740 may include the discharging circuit 714.

According to an embodiment, the first circuit 1730 may generate a second voltage VDC based on the first voltage VMOT (e.g., the voltage of the battery 720 or the voltage of the motor electromotive force) used by the motor 718. The first circuit 1730 may determine whether the motor 718 is in an overvoltage state based on at least one of the first voltage or the second voltage.

According to an embodiment, the second circuit 1740 may consume the motor electromotive force through one or more resistors. For example, when the motor 718 is in an overvoltage state, a transistor (e.g., the transistor F_BEMF 816-2) in the first circuit 1730 may be turned on, and the second circuit 1740 may consume the motor electromotive force through one or more resistors via the turned-on transistor.

According to an embodiment, the first circuit 1730 may output (or transmit) the second voltage VDC to the processor 1710. The first circuit 1730 may generate a diagnostic voltage (e.g., the diagnostic voltage V_DIAG) based on the first voltage VMOT, a resistor in the second circuit 1740, and an electrical element (e.g., the resistor R_EXT, etc.) and may output (or transmit) the generated diagnostic voltage to the processor 1710.

According to an embodiment, the processor 1710 may determine whether the second circuit 1740 is in an abnormal state based on at least one of the voltage value of the second voltage VDC or the voltage value of the diagnostic voltage.

According to an embodiment, the processor 1710 may determine whether the second circuit 1740 is in an abnormal state based on the amount of change in VDC during a time interval (e.g., the aforementioned $\Delta t$) from a first time point (e.g., the time at which the motor is determined to be in an overvoltage state or the time at which the converted voltage VDC is greater than or equal to the reference voltage) to a second time point. When determining that the motor is not in an overvoltage state, the wearable apparatus 700 may determine whether the second circuit 1740 is in an abnormal state based on the voltage value of the diagnostic voltage.

The embodiments described with reference to FIGS. 1 to 16 may apply to the embodiments of FIG. 17.

According to an embodiment, a wearable apparatus 100, 500, 500-1, 700, 1700 may include a motor 718, a conversion circuit 710 configured to generate a second voltage (e.g., VDC) based on a first voltage (e.g., VMOT) used by the motor, a discharging circuit 714 including one or more resistors and configured to consume an electromotive force generated by the motor through the resistors when the second voltage is greater than or equal to a reference voltage, a diagnostic circuit 717 including one or more electrical elements and configured to output a diagnostic voltage generated based on the first voltage, the resistors in the discharging circuit, and the electrical elements, and a processor 512, 712, 1710 configured to receive the second voltage from the conversion circuit, receive the output diagnostic voltage from the diagnostic circuit, and determine whether the discharging circuit is in an abnormal state based on at least one of a voltage value of the second voltage or a voltage value of the received diagnostic voltage.

The abnormal state may include a first abnormal state indicating that the discharging circuit is in an electrically open state or a second abnormal state indicating that the discharging circuit is in an electrically short state.

The processor may determine whether the discharging circuit is in the abnormal state based on the amount of change in the second voltage during a time interval (e.g., $\Delta t$ of FIG. 13) from a first time point (e.g., $t_1$ of FIG. 13) at which the second voltage is greater than or equal to the reference voltage to a second time point (e.g., $t_2$ of FIG. 13).

The processor may determine whether the discharging circuit is in the abnormal state based on a current value of a current flowing through the discharging circuit when the second voltage is greater than or equal to the reference voltage.

The processor may determine whether the discharging circuit is in the abnormal state based on the voltage value of the received diagnostic voltage when the second voltage is less than the reference voltage.

The processor may determine whether the voltage value of the received diagnostic voltage corresponds to a first voltage value (e.g., a normal voltage value) when a diagnostic request signal for the discharging circuit exists in the pre-exercise state of the post-exercise state of the user wearing the wearable apparatus. The processor may determine whether the voltage value of the received diagnostic voltage is greater than the first voltage value or corresponds to a second voltage value (e.g., 0) when the voltage value of the received diagnostic voltage does not correspond to the first voltage value. The processor may determine that the discharging circuit is in a first abnormal state when the voltage value of the received diagnostic voltage corresponds to the second voltage value. The processor may determine that the discharging circuit is in a second abnormal state when the voltage value of the received diagnostic voltage is greater than the first voltage value.

The processor may determine whether the discharging circuit is in the abnormal state through a change in the second voltage in an exercising state of the user wearing the wearable apparatus.

The processor may obtain the voltage value (e.g., $V_1$ of FIG. 13) of the second voltage at the first time point at which the second voltage is greater than or equal to the reference voltage. The processor may obtain the voltage value of the second voltage (e.g., $V_2$, $V_3$, or $V_4$ of FIG. 13) at the second time point after a predetermined time elapses from the first time point. The processor may determine that the discharging circuit is in the first abnormal state when the second voltage value is greater than the first voltage value. The processor may determine that the discharging circuit is in the second abnormal state when the second voltage value is less than the first voltage value by more than a predetermined level.

The processor may enable a first switch circuit 715 to be in a turned-on state such that the diagnostic circuit is electrically connected, directly or indirectly, to the discharging circuit when the second voltage is less than the reference voltage at a time point at which a diagnostic request signal for the discharging circuit exists. The processor may determine whether the discharging circuit is in an abnormal state based on the voltage value of the received diagnostic voltage. The processor may enable the first switch circuit to be in a turned-off state when the second voltage is greater than or equal to the reference voltage at a time point at which a diagnostic request signal for the discharging circuit exists. The processor may determine whether the discharging circuit is in an abnormal state based on the voltage value of the second voltage.

The diagnostic request signal may be generated based on the diagnostic request received from an electronic device 210 that is in a wireless communication connection with the wearable apparatus or generated as a period of the diagnostic request signal is reached.

The processor may control a report, which indicates that the discharging circuit is in an abnormal state, to be transmitted to at least one of a cloud server or the electronic device 210 of a user when it is determined that the discharging circuit is in an abnormal state.

The processor may control the motor to provide a torque to the user in an exercising state of the user wearing the wearable apparatus. The processor may control the motor to stop providing the torque to the user when it is determined that the discharging circuit is in an abnormal state in the exercising state.

The wearable apparatus may include one or more light-emitting diodes (LEDs) configured to output light corresponding to a state (e.g., a charging state, an abnormal state, etc.) of the wearable apparatus. The processor may control the one or more LEDs to use the electromotive force as a power source of the one or more LEDs.

The wearable apparatus may include a first switch circuit located between the discharging circuit and the diagnostic circuit and configured to connect the discharging circuit to the diagnostic circuit when the first switch circuit is in a turned-on state.

The wearable apparatus may further include a comparison circuit 711 configured to compare the second voltage with the reference voltage and a second switch circuit 716 that is in a turned-on state or a turned-off state based on a result of the comparing of the comparison circuit.

The discharging circuit may receive and consume the electromotive force when the second switch circuit is in the turned-on state.

According to an embodiment, a wearable apparatus 100, 500, 500-1, 700, 1700 may include a motor 718, a first circuit 130 including one or more electrical elements and configured to determine whether the motor is in an overvoltage state based on a first voltage used by the motor, a second circuit 1740 including one or more resistors and configured to consume an electromotive force generated by the motor through the one or more resistors, and a processor 512, 712, 1710. The first circuit may output a second voltage generated based on the first voltage to the processor and output a diagnostic voltage generated based on the first voltage, a resistor in the second circuit, and the one or more electrical elements to the processor. The processor may determine whether the second circuit is in an abnormal state based on at least one of a voltage value of the second voltage or a voltage value of the diagnostic voltage.

The abnormal state may correspond to a first abnormal state including an electrical open state of the second circuit or a second abnormal state including an electrical short state of the second circuit.

The first circuit may determine that the motor is in an overvoltage state when the second voltage is greater than or equal to a reference voltage.

The processor may determine whether the second circuit is in an abnormal state through the amount of change in the second voltage during a time interval from a first time point, at which it is determined that the motor is in an overvoltage state, to a second time point. The processor may determine whether the second circuit is in an abnormal state based on a voltage value of the diagnostic voltage when it is determined that the motor is not in the overvoltage state.

According to an embodiment, an operating method of a wearable apparatus 100, 500, 500-1, 700, 1700 may include an operation (hereinafter, "first operation") of generating a second voltage based on a first voltage used by a motor of the wearable apparatus, an operation (hereinafter, "second operation") of consuming an electromotive force generated by the motor through a discharging circuit including one or more resistors when the second voltage is greater than or equal to a reference voltage, an operation (hereinafter, "third operation") of generating a diagnostic voltage based on the first voltage, a resistor in the discharging circuit, and one or more electrical elements, and an operation (hereinafter, "fourth operation") of determining whether the discharging circuit is in an abnormal state based on at least one of a voltage value of the second voltage or a voltage value of the diagnostic voltage.

The fourth operation may include an operation of determining whether the discharging circuit is in an abnormal state through the amount of change in the second voltage during a time interval from a first time point at which the second voltage is greater than or equal to the reference voltage to a second time point.

The fourth operation may include an operation of determining whether the discharging circuit is in an abnormal state based on a current value of a current flowing through the discharging circuit when the second voltage is greater than or equal to the reference voltage.

The fourth operation may include an operation of determining whether the discharging circuit is in an abnormal state based on a voltage value of the diagnostic voltage when the second voltage is less than the reference voltage.

The operating method of the wearable apparatus may include an operation of generating a diagnostic request signal for the discharging circuit. The fourth operation may include an operation of determining whether a voltage value of the diagnostic voltage corresponds to a first voltage value when the diagnostic request signal exists, determining whether the voltage value of the diagnostic voltage is greater than the first voltage value or corresponds to a second voltage value when the voltage value of the diagnostic voltage does not correspond to the first voltage value, an operation of determining that the discharging circuit is in a first abnormal state when the voltage value of the diagnostic voltage corresponds to the second voltage value, and an operation of determining that the discharging circuit is in a second abnormal state when the voltage value of the diagnostic voltage is greater than the first voltage value.

The fourth operation may include an operation of determining whether the discharging circuit is in an abnormal state through a change in the second voltage in an exercising state of a user wearing the wearable apparatus.

The fourth operation may include an operation of enabling a first switch circuit to be in a turned-on state such that the diagnostic circuit is electrically connected, directly or indirectly, to the discharging circuit when the second voltage is less than the reference voltage at a time point at which the diagnostic request signal for the discharging exists, an operation of determining whether the discharging circuit is in an abnormal state based on the voltage value of the diagnostic voltage, an operation of enabling the first switch circuit to be in a turned-off state when the second voltage is greater than or equal to the reference voltage at a time point at which the diagnostic request signal for the discharging circuit exists, and an operation of determining whether the discharging circuit is in an abnormal state based on the voltage value of the second voltage. "Based on" as used herein covered based at least on.

Each embodiment herein may be used in combination with any other embodiment(s) described herein.

The operating method of the wearable apparatus may include an operation of transmitting a report indicating that the discharging circuit is in an abnormal state to at least one of a cloud server or an electronic device of a user when it is determined that the discharging circuit is in an abnormal state.

The operating method of the wearable apparatus may include an operation of controlling the motor to provide a torque to the user in an exercising state of the user wearing the wearable apparatus and an operation of controlling the motor to stop providing the torque to the user when it is determined that the discharging circuit is in an abnormal state in the exercising state.

The operating method of the wearable apparatus may include an operation of controlling LEDs to use the electromotive force as a power source of the LEDs.

The embodiments described herein may be implemented using a hardware component, a software component, and/or a combination thereof. A processing device may be implemented using one or more general-purpose or special-purpose computers, such as, for example, a processor, a controller and an arithmetic logic unit (ALU), a digital signal processor (DSP), a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, the processing device may include a plurality of processors, or a single processor and a single controller. In addition, different processing configurations are possible, such as parallel processors.

Each "module" herein may comprise circuitry.

The software may include a computer program, a piece of code, an instruction, or one or more combinations thereof, to independently or uniformly instruct or configure the processing device to operate as desired. Software and data may be stored in any type of machine, component, physical or virtual equipment, or computer storage medium or device capable of providing instructions or data to or being interpreted by the processing device. The software may also be distributed over network-coupled computer systems so that the software is stored and executed in a distributed fashion. The software and data may be stored in a non-transitory computer-readable recording medium.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of examples, or they may be of the kind well-known and available to those having skill in the computer software arts. Examples of non-transitory computer-readable media include magnetic media such as hard disks, floppy disks, and magnetic tape; optical media such as CD-ROM discs and/or DVDs; magneto-optical media such as floptical discs; and hardware devices that are specially configured to store and perform program instructions, such as read-only memory (ROM), random access memory (RAM), flash memory, and the like. Examples of program instructions include both machine code, such as produced by a compiler, and files containing higher-level code that may be executed by the computer using an interpreter.

The above-described hardware devices may be configured to act as one or more software modules in order to perform the operations of the above-described embodiments, or vice versa.

As described above, although the embodiments have been described with reference to the limited drawings, one of ordinary skill in the art may apply various technical modifications and variations based thereon. For example, suitable results may be achieved if the described techniques are performed in a different order, and/or if components in a described system, architecture, device, or circuit are combined in a different manner, or replaced or supplemented by other components or their equivalents. While the disclosure has been illustrated and described with reference to various embodiments, it will be understood that the various embodiments are intended to be illustrative, not limiting. It will further be understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

Therefore, other implementations, other embodiments, and equivalents of the claims are within the scope of the following claims.

**Claims**

1. A wearable apparatus (100, 500, 500-1, 700, 1700) comprising:

   a motor (718);
   a conversion circuit (710) configured to generate a second voltage based on a first voltage used by the motor;
   a discharging circuit (714) comprising one or more resistors and configured to consume an electromotive force generated by the motor through at least the one or more resistors when the second voltage is greater than or equal to a reference voltage;
   a diagnostic circuit (717) comprising one or more electrical circuit elements and configured to output a diagnostic voltage generated based on the first voltage, a resistor in the discharging circuit, and the one or more electrical circuit elements; and
   at least one processor, comprising processing circuitry, individually and/or collectively (512, 712, 1710) configured to receive the second voltage from the conversion circuit, receive the output diagnostic voltage from the diagnostic circuit, and determine whether the discharging circuit is in an abnormal state based on at least one of a voltage value of the second voltage or a voltage value of the received diagnostic voltage.

**2.** The wearable apparatus of claim 1, wherein the abnormal state corresponds to a first abnormal state comprising an electrical open state of the discharging circuit and a second abnormal state comprising an electrical short state of the discharging circuit, and

wherein the one or more electrical circuit elements comprises a resistor.

**3.** The wearable apparatus of claim 1, wherein the at least one processor is individually and/or collectively configured to determine whether the discharging circuit is in an abnormal state through at least an amount of change in the second voltage during a time interval from a first time point, at which the second voltage is greater than or equal to the reference voltage, to a second time point.

**4.** The wearable apparatus of claim 1, wherein the at least one processor is individually and/or collectively configured to determine whether the discharging circuit is in an abnormal state based on a current value of a current flowing through at least the discharging circuit when the second voltage is greater than or equal to the reference voltage.

**5.** The wearable apparatus of claim 1, wherein the at least one processor is individually and/or collectively configured to determine whether the discharging circuit is in an abnormal state based on a voltage value of the received diagnostic voltage when the second voltage is less than the reference voltage.

**6.** The wearable apparatus of claim 1, wherein the at least one processor is individually and/or collectively configured to determine whether a voltage of the received diagnostic voltage corresponds to a first voltage value when a diagnostic request signal for the discharging circuit exists in a pre-exercise state or post-exercise state of a user wearing the wearable apparatus, determine whether the voltage value of the received diagnostic voltage is greater than the first voltage value or corresponds to a second voltage value when the voltage value of the received diagnostic voltage does not correspond to the first voltage value, determine that the discharging circuit is in a first abnormal state when the voltage value of the received diagnostic voltage corresponds to the second voltage value, and determine that the discharging circuit is in a second abnormal state when the voltage value of the received diagnostic voltage is greater than the first voltage value.

**7.** The wearable apparatus of claim 1, wherein the at least one processor is individually and/or collectively configured to determine whether the discharging circuit is in an abnormal state through a change in the second voltage in an exercising state of a user wearing the wearable apparatus.

**8.** The wearable apparatus of claim 7, wherein the at least one processor is individually and/or collectively configured to obtain a voltage value of the second voltage at a first time point at which the second voltage is greater than or equal to the reference voltage, obtain the voltage value of the second voltage at a second time point after a predetermined time elapses from the first time point, determine that the discharging circuit is in a first abnormal state when the voltage value at the second time point is greater than the voltage value at the first time point, and determine that the discharging circuit is in a second abnormal state when the voltage value at the second time point is less than the voltage value at the first time point by more than a predetermined level.

**9.** The wearable apparatus of claim 1, wherein the at least one processor is individually and/or collectively configured to enable a first switch circuit to be in a turned-on state such that the diagnostic circuit is electrically connected to the discharging circuit when the second voltage is less than the reference voltage at a time point at which a diagnostic request signal for the discharging circuit exists, determine whether the discharging circuit is in an abnormal state based on the voltage value of the received diagnostic voltage, enable the first switch circuit to be in a turned-off state when the second voltage is greater than or equal to the reference voltage at a time point at which a diagnostic request signal for the discharging circuit exists, and determine whether the discharging circuit is in an abnormal state based on the voltage value of the second voltage.

**10.** The wearable apparatus of claim 9, wherein the diagnostic request signal is based on the diagnostic request received from an electronic device that is in a wireless communication connection with the wearable apparatus and/or based on as a period of the diagnostic request signal is reached.

**11.** The wearable apparatus of claim 1, wherein the at least one processor is individually and/or collectively configured to control a report, which indicates that the discharging circuit is in an abnormal state, to be transmitted to at least one of a cloud server and/or an electronic device of a user when it is determined that the discharging circuit is in an abnormal state.

12. The wearable apparatus of claim 1, wherein the at least one processor is individually and/or collectively configured to control the motor to provide a torque to the user in an exercising state of the user wearing the wearable apparatus and control the motor to stop providing the torque to the user when it is determined that the discharging circuit is in an abnormal state in the exercising state.

13. The wearable apparatus of claim 1, comprising:

one or more light-emitting diodes (LEDs) configured to output light corresponding to a state of the wearable apparatus,
wherein the at least one processor is individually and/or collectively configured to control the one or more LEDs to use the electromotive force as a power source of the one or more LEDs.

14. The wearable apparatus of claim 1, further comprising:

a first switch circuit located between at least the discharging circuit and the diagnostic circuit, and configured to connect the discharging circuit to the diagnostic circuit when the first switch circuit is in a turned-on state;
a comparison circuit configured to compare the second voltage with the reference voltage; and
a second switch circuit configured to be in a turned-on state and/or a turned-off state based on a result of the comparing of the comparison circuit,
wherein the discharging circuit is configured to receive and consume the electromotive force when the second switch circuit is in the turned-on state..

15. An operating method of a wearable apparatus (100, 500, 500-1, 700, 1700), the operating method comprising:

generating a second voltage based on a first voltage used by a motor of the wearable apparatus;
consuming an electromotive force generated by the motor through at least a discharging circuit comprising one or more resistors when the second voltage is greater than or equal to a reference voltage;
generating a diagnostic voltage based on the first voltage, a resistor in the discharging circuit, and one or more electrical circuit elements; and
determining whether the discharging circuit is in an abnormal state based on at least one of a voltage value of the second voltage or a voltage value of the diagnostic voltage.

**FIG. 1**

**FIG. 2**

**FIG. 3**

**FIG. 4**

**FIG. 5A**

500-1

510

Memory
514

Communication
module
516

530

Motor driver
circuit 532

Motor
534

Processor
512

530-1

Motor driver
circuit 532-1

Motor
534-1

First angle sensor
524

Sound output
module
550

Inertial
measurement unit
522

Input module
540

Second angle
sensor
524-1

FIG. 5B

210

Electronic device

Wearable apparatus
100

Control instruction

212

Electronic device
210

Wearable apparatus
100

Control result

Sensor data

212

FIG. 6

**FIG. 7**

**FIG. 8**

FIG. 9

**FIG. 10**

FIG. 11

FIG. 12

**FIG. 13**

**FIG. 14**

```
                    ┌─────────┐
                    │  Start  │
                    └────┬────┘
                         │              ┌1511
              ┌──────────┴──────────┐
              │ Receive user request│
              └──────────┬──────────┘
```

**FIG. 15**

- Start
- 1511 Receive user request
- 1512 VDC < VREF — No →
- 1513 VBC = LOW — No →
- 1514 V_DIAG = 0 — No →
- 1515 Generate diagnostic request signal
- 1516 V_DIAG = normal voltage value — No → 1518 V_DIAG = 0 — No → 1520 Determine that discharging circuit is in second abnormal state when V_DIAG is greater than normal voltage value
- 1517 Determine that discharging circuit is in normal state
- 1519 Determine that discharging circuit is in first abnormal state
- 1521 Is battery in abnormal state? — Yes →
- 1522 Determine that main PBA of wearable apparatus is in abnormal state
- 1523 Detect battery malfunction
- 1524 Transmit report indicating that wearable apparatus is in abnormal state to cloud server
- End

FIG. 15

**FIG. 16**

Start

1611 Provide external force to user

1612 VDC > VREF?
- No → 1619 Generate diagnostic request signal
- Yes → 1613 Check change in VDC

1614 Is VDC increased?
- Yes → 1615 Determine that discharging circuit is in first abnormal state
- No → 1616 Is decrease in VDC greater than or equal to predetermined level?
  - Yes → 1617 Determine that discharging circuit is in second abnormal state
  - No → 1618 Determine that discharging circuit is in normal state

1619 → 1620 V_DIAG = normal voltage value?
- Yes → 1618 Determine that discharging circuit is in normal state
- No → 1621 V_DIAG=0?
  - Yes → 1622 Determine that discharging circuit is in first abnormal state
  - No → 1623 Determine that discharging circuit is in second abnormal state when V_DIAG is greater than normal voltage value

1624 Stop driving motor

1625 Transmit report indicating that wearable apparatus is in abnormal state to cloud server

End

44

Wearable apparatus 1700

| Driving module 1720 | Processor 1710 | First circuit 1730 |

Second circuit 1740

# FIG. 17

## INTERNATIONAL SEARCH REPORT

| International application No. |
|---|
| **PCT/KR2024/014703** |

**A. CLASSIFICATION OF SUBJECT MATTER**

**A61H 3/00**(2006.01)i; **B25J 9/00**(2006.01)i; **G01R 31/28**(2006.01)i; **G01R 19/165**(2006.01)i; **G01R 31/34**(2006.01)i; **G01R 31/52**(2020.01)i; **G08C 17/02**(2006.01)i; **H05B 45/10**(2020.01)i

According to International Patent Classification (IPC) or to both national classification and IPC

**B. FIELDS SEARCHED**

Minimum documentation searched (classification system followed by classification symbols)

A61H 3/00(2006.01); A61H 1/02(2006.01); A63B 21/00(2006.01); B25J 9/00(2006.01); B25J 9/16(2006.01); B60L 3/00(2006.01); H02P 6/14(2006.01); H02P 7/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 웨어러블(wearable), 모터(motor), 전압(voltage), 방전(discharge), 기전력(electromotive force)

**C. DOCUMENTS CONSIDERED TO BE RELEVANT**

| Category* | Citation of document, with indication, where appropriate, of the relevant passages | Relevant to claim No. |
|---|---|---|
| A | KR 10-2023-0143613 A (WIROBOTICS INC.) 12 October 2023 (2023-10-12)<br>  See paragraph [0687]. | 1-15 |
| A | KR 10-1999-0086973 A (KABUSHIKI KAISHA TOSHIBA) 15 December 1999 (1999-12-15)<br>  See claim 1. | 1-15 |
| A | KR 10-2021-0126412 A (HYUNDAI MOTOR COMPANY et al.) 20 October 2021 (2021-10-20)<br>  See claims 1-3. | 1-15 |
| A | KR 10-1959330 B1 (SRI INTERNATIONAL) 21 March 2019 (2019-03-21)<br>  See claims 1-4. | 1-15 |
| A | JP 6205521 B1 (SHINDENGEN ELECTRIC MANUFACTURING CO., LTD.) 27 September 2017 (2017-09-27)<br>  See claims 1-6. | 1-15 |

☐ Further documents are listed in the continuation of Box C.   ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **03 January 2025** | **06 January 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/014703**

| Patent document cited in search report | | | Publication date (day/month/year) | Patent family member(s) | | | Publication date (day/month/year) |
|---|---|---|---|---|---|---|---|
| KR | 10-2023-0143613 | A | 12 October 2023 | EP | 4464465 | A1 | 20 November 2024 |
| | | | | KR | 10-2023-0091764 | A | 23 June 2023 |
| | | | | KR | 10-2023-0092715 | A | 26 June 2023 |
| | | | | KR | 10-2023-0092716 | A | 26 June 2023 |
| | | | | KR | 10-2023-0146041 | A | 18 October 2023 |
| | | | | KR | 10-2024-0114764 | A | 24 July 2024 |
| | | | | KR | 10-2680640 | B1 | 04 July 2024 |
| | | | | KR | 10-2734351 | B1 | 29 November 2024 |
| | | | | US | 2024-0238148 | A1 | 18 July 2024 |
| | | | | US | 2024-0316755 | A1 | 26 September 2024 |
| | | | | WO | 2023-113467 | A1 | 22 June 2023 |
| | | | | WO | 2023-113517 | A1 | 22 June 2023 |
| KR | 10-1999-0086973 | A | 15 December 1999 | CN | 1110594 | C | 04 June 2003 |
| | | | | CN | 1211649 | A | 24 March 1999 |
| | | | | JP | 11-128587 | A | 18 May 1999 |
| | | | | JP | 3225008 | B2 | 05 November 2001 |
| | | | | KR | 10-0315555 | B1 | 17 January 2002 |
| | | | | NZ | 331564 | A | 28 April 2000 |
| | | | | TW | 392015 | B | 01 June 2000 |
| KR | 10-2021-0126412 | A | 20 October 2021 | CN | 113511075 | A | 19 October 2021 |
| | | | | US | 11251605 | B2 | 15 February 2022 |
| | | | | US | 2021-0320492 | A1 | 14 October 2021 |
| KR | 10-1959330 | B1 | 21 March 2019 | BR | 112015022903 | A2 | 18 July 2017 |
| | | | | CA | 2904365 | A1 | 25 September 2014 |
| | | | | CA | 2904365 | C | 28 November 2017 |
| | | | | CN | 205521410 | U | 31 August 2016 |
| | | | | CN | 206123637 | U | 26 April 2017 |
| | | | | CN | 206123638 | U | 26 April 2017 |
| | | | | EP | 2969402 | A2 | 20 January 2016 |
| | | | | EP | 2969402 | B1 | 11 October 2017 |
| | | | | JP | 2016-521212 | A | 21 July 2016 |
| | | | | JP | 2017-196735 | A | 02 November 2017 |
| | | | | JP | 2020-073296 | A | 14 May 2020 |
| | | | | JP | 6158414 | B2 | 05 July 2017 |
| | | | | JP | 6639443 | B2 | 05 February 2020 |
| | | | | KR | 10-1779100 | B1 | 18 September 2017 |
| | | | | KR | 10-1874969 | B1 | 05 July 2018 |
| | | | | KR | 10-2015-0131350 | A | 24 November 2015 |
| | | | | KR | 10-2017-0106650 | A | 21 September 2017 |
| | | | | KR | 10-2018706 | B1 | 05 September 2019 |
| | | | | KR | 10-2019-0031584 | A | 26 March 2019 |
| | | | | US | 10105839 | B2 | 23 October 2018 |
| | | | | US | 10906168 | B2 | 02 February 2021 |
| | | | | US | 2014-0277739 | A1 | 18 September 2014 |
| | | | | US | 2015-0266180 | A1 | 24 September 2015 |
| | | | | US | 2015-0266181 | A1 | 24 September 2015 |
| | | | | US | 2016-0101516 | A1 | 14 April 2016 |
| | | | | US | 2016-0101517 | A1 | 14 April 2016 |
| | | | | US | 2018-0361566 | A1 | 20 December 2018 |
| | | | | US | 2021-0387327 | A1 | 16 December 2021 |

Form PCT/ISA/210 (patent family annex) (July 2022)

INTERNATIONAL SEARCH REPORT
Information on patent family members

International application No.

**PCT/KR2024/014703**

| Patent document cited in search report | | | Publication date (day/month/year) | Patent family member(s) | | | Publication date (day/month/year) |
|---|---|---|---|---|---|---|---|
| | | | | US | 9266233 | B2 | 23 February 2016 |
| | | | | US | 9403272 | B2 | 02 August 2016 |
| | | | | US | 9427864 | B2 | 30 August 2016 |
| | | | | US | 9950422 | B2 | 24 April 2018 |
| | | | | WO | 2014-151065 | A2 | 25 September 2014 |
| | | | | WO | 2014-151065 | A3 | 30 October 2014 |
| | | | | WO | 2014-151065 | A9 | 24 December 2014 |
| JP | 6205521 | B1 | 27 September 2017 | JP | 2018-037528 | A1 | 23 August 2018 |
| | | | | WO | 2018-037528 | A1 | 01 March 2018 |

Form PCT/ISA/210 (patent family annex) (July 2022)